(19)

Europäisches
Patentamt
European
Patent Office
Office européen
des brevets

(11) **EP 2 468 900 A2**

(12) **EUROPEAN PATENT APPLICATION**

(43) Date of publication:
   **27.06.2012 Bulletin 2012/26**

(51) Int Cl.:
   **C12Q 1/68** (2006.01)

(21) Application number: **12154714.5**

(22) Date of filing: **21.08.2008**

---

(84) Designated Contracting States:
   **AT BE BG CH CY CZ DE DK EE ES FI FR GB GR HR HU IE IS IT LI LT LU LV MC MT NL NO PL PT RO SE SI SK TR**
   Designated Extension States:
   **AL BA MK RS**

(30) Priority: **21.08.2007 US 894546**

(62) Document number(s) of the earlier application(s) in accordance with Art. 76 EPC:
   **08798395.3 / 2 191 427**

(71) Applicant: **Merial Limited**
   **Duluth, GA 30096 (US)**

(72) Inventor: **Lusk, Jayson Lee**
   **Stillwater, OK Oklahoma 74074 (US)**

(74) Representative: **Harding, Charles Thomas**
   **D Young & Co LLP**
   **120 Holborn**
   **London EC1N 2DY (GB)**

Remarks:
   This application was filed on 09-02-2012 as a divisional application to the application mentioned under INID code 62.

---

(54) **Method for predicting the carcass quality of a bovine animal**

(57)   The present invention is directed to methods and systems for improving the efficiency of livestock production using genetic information obtained from the animal. The methods of the invention comprise obtaining a genetic sample from an animal or embryo, determining the genotype of the animal or embryo with respect to specific quality traits, grouping animals with like genotypes, and optionally, further sub-grouping animals based on like phenotypes. The invention is further directed to a method of predicting the carcass quality of an animal by correlating the rate of change in carcass traits with the genotype of the animal. In particular the genotype in the leptin genes.

EP 2 468 900 A2

**Description**

INCORPORATION BY REFERENCE

This application claims benefit of U.S. patent application Serial No. 11/894546 filed August 21, 2007.
All documents cited or referenced herein ("herein cited documents"), and all documents cited or referenced in herein cited documents, together with any manufacturer's instructions, descriptions, product specifications, and product sheets for any products mentioned herein or in any document incorporated by reference herein, are hereby incorporated herein by reference, and may be employed in the practice of the invention.

FIELD OF THE INVENTION

The present invention relates to methods of predicting a marketing method for livestock that maximizes revenue based upon leptin ob gene single nucleotide polymorphisms and the association of these markers with certain economically important carcass traits such as circulating leptin levels, feed intake, growth rate, body weight, carcass merit and carcass composition. The invention further relates to a pair of SNPs, one located in the promoter of leptin gene promoter and the other within the leptin-encoding region of the *ob* gene, which is strongly associated with multiple economically important characters.

BACKGROUND OF THE INVENTION

Animals account for almost 20 percent of the world's food consumption. It follows, then, that animal-based food products are a major source of revenue throughout the world. In the United States alone, beef production is the fourth largest manufacturing industry and accounts for nearly 25 percent of the farm sector cash receipts and seven percent of supermarket sales each year.
Body condition of the animals is a determinant of market readiness in commercial livestock feeding and finishing operations. The term "body condition" is used in the livestock industry in reference to the state of development of a livestock animal that is a function of frame type or size, and the amount of intramuscular fat and back fat exhibited by an animal. It is typically determined subjectively and through experienced visual appraisal of live animals. The fat deposition, or the amount of intramuscular fat and back fat on an animal carcass, is important to industry participants because carcasses exhibiting desired amounts and proportions of such fats can often be sold for higher prices than carcasses that exhibit divergences from such desired amounts and proportions. Furthermore, the desired carcass fat deposition often varies among different markets and buyers, and it also often varies with time within single markets and among particular buyers in response to public demand trends with respect to desired fat and marbling in meats.
Presently, cattle entering a feedlot may be divided into groups according to estimated age, frame size, breed, weight, and so forth. By making such a division, the feedlot owner is attempting to group the cattle so that the group can be penned together and fed the same ration and will be ready for slaughter at the same time. There still exists, however, a need in the industry for objectively grouping livestock entering a feedlot.
The feedlot operator's costs include operations costs for the lot, such as labor, health costs, transport, animal death, capital, maintenance, and the like, plus the cost of feeding the animals. While the cost of acquiring each animal in a group can vary somewhat, the feedlot operator's costs are the same for each animal in the group since they are fed the same amount of feed and occupy space in the feedlot for the same amount of time. Thus, the price reductions for carcasses falling outside the desirable range fall directly to the feedlot operator's bottom line, resulting in reduced profits.
One way for the feedlot operator to reduce costs and increase profits is to alter the time an animal spends on the lot, thus reducing the feed costs. Longer residence times are usually only profitable if the result is an animal with a more profitable grade. Because the residence time and the feed schedule are based on visual inspections, there is no totally reliable way to accurately predict which animals will result in higher grades of meat. Similarly, meat packers predict the carcass grade based on visual inspection of the live animals, and after slaughter. It is common that the post-slaughter inventory of a specific, desired grade of meat does not meet with a packer's pre-slaughter demand. This results in an uncertain inventory, and often means that the packer must purchase additional animals to fill a desired inventory. Accordingly, there exists a need in the art for accurately and objectively predicting animal meat grades in advance of slaughter. Additionally, methods and systems are needed for tracking individual animals easily and accurately and maintaining data associated with individual animals.
The increased emphasis on value-based marketing has also forced producers to consider the quality of their cattle prior to making marketing decisions. Incorrect marketing choices can lead to substantial losses in income. Although there is an increasing need for producers to know the quality of their cattle prior to slaughter, they currently have little objective information on which to make these judgments. Genetic testing provides a way for producers to potentially obtain information about carcass characteristics prior to slaughter.

There is a need for methods that allow relatively easy and more efficient selection and breeding of farm animals with an advantage for an inheritable trait of circulating leptin levels, feed intake, growth rate, body weight, carcass merit and carcass composition. The economic significance of the use of genetic markers that are associated with specific economically important traits (especially traits with low heritability) in livestock through marker-assisted selection cannot therefore be over-emphasized.

Suitable genetic markers with potential association with economically significant phenotypic characters include leptin, the hormone product of the ob (obese) gene, has been shown to be predominantly synthesized and expressed in adipose tissues (Zhang et al. Nature 372: 425-432 (1994); Ji et al. Anim. Biotech. 9: 1-14 (1998)). It functions as a potent physiological signal in the regulation of body weight, energy expenditure, feed intake, adiposity, fertility and immune functions (Houseknecht et al. J. Anim. Sci. 76: 1405-1420 (1998); Lord et al. Nature 394: 897-901 (1998); Williams et al. Domest. Anim. Endocrinol. 23: 339-349 (2002)). Leptin has been proposed as one of the major control factors contributing to the phenotypic and genetic variation in the performance and efficiency of cattle.

Polymorphisms in the coding regions of the leptin gene in cattle have been associated with milk yield and composition (Liefers et al. J. dairy Sci. 85: 1633-1638 (2002), feed intake (Liefers et al., (2002); Lagonigro et al. Anim. Genet. 34: 371-374 (2003), and body fat (Buchanan et al. Genet Sel. Evol. 34: 105-116 (2002); Lagonigro et al., (2003)). However, it would appear that polymorphisms located in the promoter region of the leptin gene (i.e. the region of the gene that regulates the level of leptin expression through its associated enhancer and silencer elements) may have a stronger effect on the regulation of these economically important traits, and therefore be of greater predictive value.

There remains a need, however, for more refined genetic markers associated with economically significant characters of livestock animals, especially of cattle. There is also an unmet need for methods for predicting the animal quality, especially meat quality after feeding based upon a determination of the animal's genotype, preferably before it enters a feedlot. Such methods would allow selection of animals, based upon their genotype and predicted meat quality and market pricing, that provide the maximum revenue yield, and to select the most profitable marketing method for a an individual or group of animals.

## SUMMARY OF THE INVENTION

Citation or identification of any document in this application is not an admission that such document is available as prior art to the present invention.

The present invention is directed to computer-assisted methods and systems for improving the efficiency of livestock production by using genetic information to predict the revenue outcome of possible marketing methods. Methods of the invention also encompasses obtaining a genetic sample from each animal in a herd of livestock, determining the genotype of each animal, grouping animals with like genotypes, and optionally, further sub-grouping animals based on like phenotypes. In particular, the present invention uses pairs of single nucleotide polymorphisms (SNPs) in the promoter region of the leptin ob gene and one SNP in exon 2 of the leptin gene that are strongly associated with several economically important traits in cattle.

The methods encompassed by the present invention determine the best marketing method, either live weight, dressed weight, or grid basis, for cattle of different genotypes. The invention provides regression models for predicting the yield grade, quality grade, and dressing percentage of an animal given its genotype and other background information and which together provide an interactive tool for cattle producers to determine the types of cattle to purchase and how to optimally to market them.

The methods according to the present invention provide estimated prediction equations useful in determining an animal's expected yield grade, marbling score, and dressing percentage. The predictive equations may be used to determine and compare the value of using genetic information to improve marketing decisions. Based on these predicted values, the expected revenue from each marketing method (live weight, dressed weight, and grid) may then be calculated. Each animal may then be targeted to the marketing method that offers the highest *expected* revenue given the expected carcass characteristics.

The following steps are used to calculate revenue from this method: (a) determining live weight price, dressed weight price, and grid premiums and discounts according to predetermined statistical data; (b) predicting the yield grade, quality grade, and dressing percentage using the estimated prediction equations; (c) given the carcass predictions and predefined market prices, calculating the expected revenue for live weight, dressed weight, and grid marketing methods; (d) marketing each animal by the method that produces the greatest expected revenue; and (e) calculating the actual revenue, using actual carcass characteristics and predefined prices, when animals are marketed in the marketing manner described by step (d).

In one embodiment of the present invention, the homozygosity or heterozygosity of each animal is determined with respect to alleles of one or more genes, most advantageously the leptin (ob) gene, encoding a trait-specific polypeptide or a control function, or other identifying nucleic acid sequences. Animals determined to be homozygous for a genetic marker associated with a particular trait, are grouped with other like-homozygous animals and are segregated from dissimilar animals. Each group may then be fed according to a regimen and for a period of time designed to optimize the desired traits in the animal while efficiently managing the resources of the livestock producer.

An advantageous aspect of the present invention is directed to a computer system and computer-assisted method for predicting quality traits for livestock possessing specific genetic predispositions. In this method, an animal is genotyped, advantageously based on SNPs of the *ob* gene and in particular the UASMS2 and EXON2-FB SNPs, and most advantageously of the UASMS2-EXON2-FB pair of SNPS. The animal may then be segregated with like-genotype animals, and bred or fed according to regimens specific to optimize the traits or characteristics. In another embodiment, a farmer can maximize feeding regimen efficiencies based on genetic predispositions. In a further embodiment, a meat packer or other commercial purchaser can base his purchase of livestock on the results of genotyping.

The present invention further provides computer-assisted methods for genotyping animals, collecting and storing the data resulting from genotyping, classifying livestock based on the genetic data, and establishing feed and slaughter schedules for livestock possessing like genetic traits based upon the predictive models determining those marketing methods that provide the maximum revenue for each identified genotype. The methods of the present invention optimize the efficiencies of raising livestock because the producer or packer can predict optimum slaughter schedules for each animal, based on the animal's genetically determined predisposition to desired end-product characteristics.

One aspect of the invention is a computer-assisted method for determining revenue from a cattle marketing method comprising using a programmed computer comprising a processor, a data storage system, an input device and an output device, and the steps of: (a) determining the genotype of an animal or group of animals by identifying at least two single length polymorphisms of the animal or animals; (b) inputting data into the programmed computer through the input device, wherein the data comprises a genotype of an animal, a physical characteristic of the animal at placement, a carcass prediction and a plurality of predefined market prices; (c) calculating a revenue expectation from a cattle marketing method for a plurality of genotypes by calculating a live weight price, a dressed weight price, and a grid price for each genotype, wherein the grid price comprises premium and discount prices; (d) correlating the expected revenues with the genotypes and the marketing methods; and (e) outputting to the output device the expected revenues for the cattle marketing methods.

In one embodiment of this aspect of the invention, the method further comprises the step of identifying the marketing method providing the highest revenue for an animal or group of animals.

In another embodiment of this aspect of the invention, the method further comprises the step of identifying the marketing method providing the highest revenue for a genotype.

In the various embodiments of this aspect of the invention, the input data may be selected from a genotype, placement weight, ultrasound backfat measurement at placement, frame score at placement, days on feed, and gender and the like.

In other embodiments of the invention, the method may further comprise the steps of (a) calculating the predicted values of the yield grade, the quality grade, and the dressing percentage of an animal for the cattle marketing method by using at least one estimated prediction equation defining the change in a trait of an animal over a period of feeding; (b) calculating the expected revenues for live weight, dressed weight and grid marketing methods by using the predicted values of yield grade, quality grade, and dressing percentage; and (c) determining the marketing method giving the highest expected revenue for the animal.

In various embodiments of the present invention, a trait of an animal predicted by an equation is selected from the group of traits consisting of backfat production, marbling score, weight prediction, dressing percentage, dry matter intake and rib eye area.

In one embodiment of the invention, the method of calculating the predicted yield grade, quality grade, and dressing percentage values of an animal for the cattle marketed is by using a plurality of prediction equations, wherein the predictive equations determine the changes in a plurality of traits of an animal over a feeding period.

In various embodiments of the invention, the genotype of the animal is determined from at least two single-length polymorphisms of the *ob* gene, thereby defining a genotype of the subject animal(s). In one embodiment of the invention, the single-length polymorphisms are UASMS2 and EXON2-FB of the *ob* gene.

In yet another embodiment of the invention, the method further comprises the step of marketing each animal by the marketing method that produces the greatest expected revenue.

In the embodiments of the invention, the genotype may be an ob genotype, wherein the physical characteristic correlating to a CC genotype is a low propensity to deposit fat, the physical characteristic correlating to a TT genotype is a high propensity to deposit fat and the physical characteristic correlating to a CT genotype is an intermediate propensity to deposit fat.

The invention further encompasses embodiments, wherein the methods further comprise the step of transmitting data, said step comprising transmission of information from the computer-based systems of the invention via telecommunication, telephone, video conference, mass communication, presentation graphics, internet, email, or paper or electronic documentary communication.

The present invention therefore encompasses a computer-assisted method for determining revenue from a cattle marketing method comprising using a programmed computer, said programmed computer comprising a processor, a data storage system, an input device and an output device, and the steps of: (a) determining the genotype of an animal or group of animals by identifying the genootype of the animal or animals, wherein single-length polymorphisms defining the genotype are UASMS2 and EXON2-FB of the *ob* gene; (b) inputting data into the programmed computer through the input device, wherein the data comprises a genotype of an animal and at least one physical characteristic of the animal at placement, a carcass prediction and a plurality of predefined market prices, genotype, placement weight, ultrasound backfat measurement at placement, frame score at placement, days on feed, and gender; (c) calculating a revenue expectation from a cattle marketing method for a plurality of genotypes by calculating a live weight price, a dressed weight price, and a grid price for each genotype, wherein the grid price comprises premium and discount prices; (d) correlating the expected revenues with the genotypes and the marketing methods; (e) calculating the predicted values of the yield grade, the quality grade, and the dressing percentage of an animal for the cattle marketing method by using a plurality of estimated prediction equations, wherein the predictive equations determine the changes in a plurality of traits of an animal over a feeding period, and wherein the traits of an animal are selected from the group of traits consisting of backfat production, marbling score, weight, dressing percentage, dry matter intake and rib eye area; (f) calculating the expected revenues for live weight, dressed weight and grid marketing methods by using the predicted values of yield grade, quality grade, and dressing percentage; (g) determining the marketing method giving the highest predicted expected revenue for the animal (h) identifying the marketing method providing the highest revenue for an animal or group of animals; (i) outputting to the output device the expected revenues for the cattle marketing methods; and (j) marketing each animal by the marketing method that produces the greatest expected revenue.

It is noted that in this disclosure and particularly in the claims and/or paragraphs, terms such as "comprises", "comprised", "comprising" and the like can have the meaning attributed to it in U.S. Patent law; e.g., they can mean "includes", "included", "including", and the like; and that terms such as "consisting essentially of" and "consists essentially of" have the meaning ascribed to them in U.S. Patent law, e.g., they allow for elements not explicitly recited, but exclude elements that are found in the prior art or that affect a basic or novel characteristic of the invention.

These and other embodiments are disclosed or are obvious from and encompassed by, the following Detailed Description.

## BRIEF DESCRIPTION OF THE DRAWING

The following detailed description, given by way of example, but not intended to limit the invention solely to the specific embodiments described, may best be understood in conjunction with the accompanying drawings, in which:

Fig. 1 illustrates the genotype differences for Hot Carcass Weight estimated from haplotypes as seen with the UASMS2 & EXON2-FB marker combination.
Fig. 2 illustrates the genotype differences for Ribeye Area estimated from haplotypes as seen with the UASMS2 & EXON2-FB marker combination.
Fig. 3 illustrates the genotype differences for Backfat estimated from haplotypes as seen with the UASMS2 & EXON2-FB marker combination.
Fig. 4 illustrates the genotype differences for Yield Grade estimated from haplotypes as seen with the UASMS2 & EXON2-FB marker combination.
Fig. 5 illustrates the genotype differences for Dressing Percentage estimated from haplotypes as seen with the UASMS2 & EXON2-FB marker combination.
Fig. 6 illustrates the genotype differences for Marbling estimated from haplotypes as seen with the UASMS2 & EXON2-FB marker combination.
Fig. 7 illustrates the estimation results of the backfat prediction model.
Fig. 8 illustrates the estimation results of the marbling score prediction model.
Fig. 9 illustrates the estimation results of the live weight prediction model.
Fig. 10 illustrates the estimation results of the dressing percentage prediction model.
Fig. 11 illustrates the estimation results of the dry matter intake prediction model.
Fig. 12 illustrates the estimation results of the rib eye area prediction model.
Fig. 13 illustrates the predicted outcomes for the three marketing methods of live weight, dressed and grid for a group ofE-CC/U-CC cattle in a simulated feeding

## DETAILED DESCRIPTION

### *Definition*

In the description that follows, a number of terms are extensively utilized. In order to provide a clear and consistent understanding of the specification and claims, including the scope to be given such terms, the following terminology is provided:

The term "animal" is used herein to include all vertebrate animals, including humans. It also includes an individual animal in all stages of development, including embryonic and fetal stages. As used herein, the term "production animals" is used interchangeably with "livestock animals" and refers generally to animals raised primarily for food. For example, such animals include, but are not limited to, cattle (bovine), sheep (ovine), pigs (porcine or swine), poultry (avian), and the like. As used herein, the term "cow" or "cattle" is used generally to refer to an animal of bovine origin of any age. Interchangeable terms include "bovine," "calf," "steer," "bull," "heifer" and the like. As used herein, the term "pig" or "swine" is used generally to refer to an animal of porcine origin of any age. Interchangeable terms include "piglet," "sow" and the like.

By the term "complementarity" or "complementary" is meant, for the purposes of the specification or claims, a sufficient number in the oligonucleotide of complementary base pairs in its sequence to interact specifically (hybridize) with the target nucleic acid sequence of the *ob* gene polymorphism to be amplified or detected. As known to those skilled in the art, a very high degree of complementarity is needed for specificity and sensitivity involving hybridization, although it need not be 100%. Thus, for example, an oligonucleotide that is identical in nucleotide sequence to an oligonucleotide disclosed herein, except for one base change or substitution, may function equivalently to the disclosed oligonucleotides. A "complementary DNA" or "cDNA" gene includes recombinant genes synthesized by reverse transcription of messenger RNA ("mRNA").

A "cyclic polymerase-mediated reaction" refers to a biochemical reaction in which a template molecule or a population of template molecules is periodically and repeatedly copied to create a complementary template molecule or complementary template molecules, thereby increasing the number of the template molecules over time.

"Denaturation" of a template molecule refers to the unfolding or other alteration of the structure of a template so as to make the template accessible to duplication. In the case of DNA, "denaturation" refers to the separation of the two complementary strands of the double helix, thereby creating two complementary, single stranded template molecules. "Denaturation" can be accomplished in any of a variety of ways, including by heat or by treatment of the DNA with a base or other denaturant.

A "detectable amount of product" refers to an amount of amplified nucleic acid that can be detected using standard laboratory tools. A "detectable marker" refers to a nucleotide analog that allows detection using visual or other means. For example, fluorescently labeled nucleotides can be incorporated into a nucleic acid during one or more steps of a cyclic polymerase-mediated reaction, thereby allowing the detection of the product of the reaction using, e.g. fluorescence microscopy or other fluorescence-detection instrumentation.

By the term "detectable moiety" is meant, for the purposes of the specification or claims, a label molecule (isotopic or non-isotopic) which is incorporated indirectly or directly into an oligonucleotide, wherein the label molecule facilitates the detection of the oligonucleotide in which it is incorporated, for example when the oligonucleotide is hybridized to amplified ob gene polymorphism sequences. Thus, "detectable moiety" is used synonymously with "label molecule." Synthesis of oligonucleotides can be accomplished by any one of several methods known to those skilled in the art. Label molecules, known to those skilled in the art as being useful for detection, include chemiluminescent or fluorescent molecules. Various fluorescent molecules are known in the art which are suitable for use to label a nucleic acid for the method of the present invention. The protocol for such incorporation may vary depending upon the fluorescent molecule used. Such protocols are known in the art for the respective fluorescent molecule.

By "detectably labeled" is meant that a fragment or an oligonucleotide contains a nucleotide that is radioactive, or that is substituted with a fluorophore, or that is substituted with some other molecular species that elicits a physical or chemical response that can be observed or detected by the naked eye or by means of instrumentation such as, without limitation, scintillation counters, colorimeters, UV spectrophotometers and the like. As used herein, a "label" or "tag" refers to a molecule that, when appended by, for example, without limitation, covalent bonding or hybridization, to another molecule, for example, also without limitation, a polynucleotide or polynucleotide fragment, provides or enhances a means of detecting the other molecule. A fluorescence or fluorescent label or tag emits detectable light at a particular wavelength when excited at a different wavelength. A radiolabel or radioactive tag emits radioactive particles detectable with an instrument such as, without limitation, a scintillation counter. Other signal generation detection methods include: chemiluminescence, electrochemiluminescence, raman, colorimetric, hybridization protection assay, and mass spectrometry.

"DNA amplification" as used herein refers to any process that increases the number of copies of a specific DNA sequence by enzymatically amplifying the nucleic acid sequence. A variety of processes are known. One of the most commonly used is the polymerase chain reaction (PCR), which is defined and described in later sections below. The PCR process of Mullis is described in U.S. Pat. Nos. 4,683,195 and 4,683,202. PCR involves the use of a thermostable DNA polymerase, known sequences as primers, and heating cycles, which separate the replicating deoxyribonucleic acid (DNA), strands and exponentially amplify a gene of interest. Any type of PCR, such as quantitative PCR, RT-PCR, hot start PCR, LAPCR, multiplex PCR, touchdown PCR, *etc.,* may be used. Advantageously, real-time PCR is used. In general, the PCR amplification process involves an enzymatic chain reaction for preparing exponential quantities of a specific nucleic acid sequence. It requires a small amount of a sequence to initiate the chain reaction and oligonucleotide primers that will hybridize to the sequence. In PCR the primers are annealed to denatured nucleic acid followed by extension with an inducing agent (enzyme) and nucleotides. This results in newly synthesized extension products. Since these newly synthesized sequences become templates for the primers, repeated cycles of denaturing, primer annealing, and extension results in exponential accumulation of the specific sequence being amplified. The extension product of the chain reaction will be a discrete nucleic acid duplex with a termini corresponding to the ends of the specific primers employed.

"DNA" refers to the polymeric form of deoxyribonucleotides (adenine, guanine, thymine, or cytosine) in either single stranded form, or as a double-stranded helix. This term refers only to the primary and secondary structure of the molecule, and does not limit it to any particular tertiary forms. Thus, this term includes double-stranded DNA found, *inter alia,* in linear DNA molecules (*e.g.,* restriction fragments), viruses, plasmids, and chromosomes. In discussing the structure of particular double-stranded DNA molecules, sequences may be described herein according to the normal convention of giving only the sequence in the 5' to 3' direction along the nontranscribed strand of DNA (*i.e.,* the strand having a sequence homologous to the mRNA).

By the terms "enzymatically amplify" or "amplify" is meant, for the purposes of the specification or claims, DNA amplification, i.e., a process by which nucleic acid sequences are amplified in number. There are several means for enzymatically amplifying nucleic acid sequences. Currently the most commonly used method is the polymerase chain reaction (PCR). Other amplification methods include LCR (ligase chain reaction) which utilizes DNA ligase, and a probe consisting of two halves of a DNA segment that is complementary to the sequence of the DNA to be amplified, enzyme QB replicase and a ribonucleic acid (RNA) sequence template attached to a probe complementary to the DNA to be copied which is used to make a DNA template for exponential production of complementary RNA; strand displacement amplification (SDA); Qß replicase amplification (QßRA); self-sustained replication (3SR); and NASBA (nucleic acid sequence-based amplification), which can be performed on RNA or DNA as the nucleic acid sequence to be amplified.

A "fragment" of a molecule such as a protein or nucleic acid is meant to refer to any portion of the amino acid or nucleotide genetic sequence.

As used herein, the term "genome" refers to all the genetic material in the chromosomes of a particular organism. Its size is generally given as its total number of base pairs. Within the genome, the term "gene" refers to an ordered sequence of nucleotides located in a particular position on a particular chromosome that encodes a specific functional product (*e.g.*, a protein or RNA molecule). For example, it is known that the protein leptin is encoded by the *ob* (obese) gene and appears to be involved in the regulation of appetite, basal metabolism and fat deposition. In general, an animal's genetic characteristics, as defined by the nucleotide sequence of its genome, are known as its "genotype," while the animal's physical traits are described as its "phenotype."

By "heterozygous" or "heterozygous polymorphism" is meant that the two alleles of a diploid cell or organism at a given locus are different, that is, that they have a different nucleotide exchanged for the same nucleotide at the same place in their sequences.

By "homozygous" or "homozygous polymorphism" is meant that the two alleles of a diploid cell or organism at a given locus are identical, that is, that they have the same nucleotide for nucleotide exchange at the same place in their sequences.

By "hybridization" or "hybridizing," as used herein, is meant the formation of A-T and C-G base pairs between the nucleotide sequence of a fragment of a segment of a polynucleotide and a complementary nucleotide sequence of an oligonucleotide. By complementary is meant that at the locus of each A, C, G or T (or U in a ribonucleotide) in the fragment sequence, the oligonucleotide sequenced has a T, G, C or A, respectively. The hybridized fragment/oligonucleotide is called a "duplex."

A "hybridization complex," such as in a sandwich assay, means a complex of nucleic acid molecules including at least the target nucleic acid and a sensor probe. It may also include an anchor probe.

By "immobilized on a solid support" is meant that a fragment, primer or oligonucleotide is attached to a substance at a particular location in such a manner that the system containing the immobilized fragment, primer or oligonucleotide may be subjected to washing or other physical or chemical manipulation without being dislodged from that location. A number of solid supports and means of immobilizing nucleotide-containing molecules to them are known in the art; any of these supports and means may be used in the methods of this invention.

As used herein, the term "increased weight gain" means a biologically significant increase in weight gain above the mean of a given population.

As used herein, the term "locus" or "loci" refers to the site of a gene on a chromosome. A single allele from each locus is inherited from each parent. Each animal's particular combination of alleles is referred to as its "genotype". Where both alleles are identical, the individual is said to be homozygous for the trait controlled by that pair of alleles; where the alleles are different, the individual is said to be heterozygous for the trait.

A "melting temperature" is meant the temperature at which hybridized duplexes dehybridize and return to their single-stranded state. Likewise, hybridization will not occur in the first place between two oligonucleotides, or, herein, an oligonucleotide and a fragment, at temperatures above the melting temperature of the resulting duplex. It is presently advantageous that the difference in melting point temperatures of oligonucleotide-fragment duplexes of this invention be from about 1°C to about 10°C so as to be readily detectable.

As used herein, the term "nucleic acid molecule" is intended to include DNA molecules (*e.g.*, cDNA or genomic DNA), RNA molecules (*e.g.*, mRNA), analogs of the DNA or RNA generated using nucleotide analogs, and derivatives, fragments and homologs thereof. The nucleic acid molecule can be single-stranded or double-stranded, but advantageously is double-stranded DNA. An "isolated" nucleic acid molecule is one that is separated from other nucleic acid molecules that are present in the natural source of the nucleic acid. A "nucleoside" refers to a base linked to a sugar. The base may be adenine (A), guanine (G) (or its substitute, inosine (I)), cytosine (C), or thymine (T) (or its substitute, uracil (U)). The sugar may be ribose (the sugar of a natural nucleotide in RNA) or 2-deoxyribose (the sugar of a natural nucleotide in DNA). A "nucleotide" refers to a nucleoside linked to a single phosphate group.

As used herein, the term "oligonucleotide" refers to a series of linked nucleotide residues, which oligonucleotide has a sufficient number of nucleotide bases to be used in a PCR reaction. A short oligonucleotide sequence may be based on, or designed from, a genomic or cDNA sequence and is used to amplify, confirm, or reveal the presence of an identical, similar or complementary DNA or RNA in a particular cell or tissue. Oligonucleotides may be chemically synthesized and may be used as primers or probes. Oligonucleotide means any nucleotides of more than 3 bases in length used to facilitate detection or identification of a target nucleic acid, including probes and primers.

"Polymerase chain reaction" or "PCR," refers to a thermocyclic, polymerase-mediated, DNA amplification reaction. A PCR typically includes template molecules, oligonucleotide primers complementary to each strand of the template molecules, a thermostable DNA polymerase, and deoxyribonucleotides, and involves three distinct processes that are multiply repeated to effect the amplification of the original nucleic acid. The three processes (denaturation, hybridization, and primer extension) are often performed at distinct temperatures, and in distinct temporal steps. In many embodiments, however, the hybridization and primer extension processes can be performed concurrently. The nucleotide sample to be analyzed may be PCR amplification products provided using the rapid cycling techniques described in U.S. Pat. Nos. 6,569,672; 6,569,627; 6,562,298; 6,556,940; 6,569,672; 6,569,627; 6,562,298; 6,556,940; 6,489,112; 6,482,615; 6,472,156; 6,413,766; 6,387,621; 6,300,124; 6,270,723; 6,245,514; 6,232,079; 6,228,634; 6,218,193; 6,210,882; 6,197,520; 6,174,670; 6,132,996; 6,126,899; 6,124,138; 6,074,868; 6,036,923; 5,985,651; 5,958,763; 5,942,432; 5,935,522; 5,897,842; 5,882,918; 5,840,573; 5,795,784; 5,795,547; 5,785,926; 5,783,439; 5,736,106; 5,720,923; 5,720,406; 5,675,700; 5,616,301; 5,576,218 and 5,455,175, the disclosures of which are incorporated by reference in their entireties. Other methods of amplification include, without limitation, NASBR, SDA, 3SR, TSA and rolling circle replication. It is understood that, in any method for producing a polynucleotide containing given modified nucleotides, one or several polymerases or amplification methods may be used. The selection of optimal polymerization conditions depends on the application.

A "polymerase" is an enzyme that catalyzes the sequential addition of monomeric units to a polymeric chain, or links two or more monomeric units to initiate a polymeric chain. In advantageous embodiments of this invention, the "polymerase" will work by adding monomeric units whose identity is determined by and which is complementary to a template molecule of a specific sequence. For example, DNA polymerases such as DNA pol 1 and Taq polymerase add deoxyribonucleotides to the 3' end of a polynucleotide chain in a template-dependent manner, thereby synthesizing a nucleic acid that is complementary to the template molecule. Polymerases may be used either to extend a primer once or repetitively or to amplify a polynucleotide by repetitive priming of two complementary strands using two primers.

A "polynucleotide" refers to a linear chain of nucleotides connected by a phosphodiester linkage between the 3'-hydroxyl group of one nucleoside and the 5'-hydroxyl group of a second nucleoside which in turn is linked through its 3'-hydroxyl group to the 5'-hydroxyl group of a third nucleoside and so on to form a polymer comprised of nucleosides liked by a phosphodiester backbone. A "modified polynucleotide" refers to a polynucleotide in which one or more natural nucleotides have been partially or substantially replaced with modified nucleotides.

A "primer" is an oligonucleotide, the sequence of at least a portion of which is complementary to a segment of a template DNA which to be amplified or replicated. Typically primers are used in performing the polymerase chain reaction (PCR). A primer hybridizes with (or "anneals" to) the template DNA and is used by the polymerase enzyme as the starting point for the replication/amplification process. By "complementary" is meant that the nucleotide sequence of a primer is such that the primer can form a stable hydrogen bond complex with the template; *i.e.*, the primer can hybridize or anneal to the template by virtue of the formation of base-pairs over a length of at least ten consecutive base pairs.

The primers herein are selected to be "substantially" complementary to different strands of a particular target DNA sequence. This means that the primers must be sufficiently complementary to hybridize with their respective strands. Therefore, the primer sequence need not reflect the exact sequence of the template. For example, a non-complementary nucleotide fragment may be attached to the 5' end of the primer, with the remainder of the primer sequence being complementary to the strand. Alternatively, non-complementary bases or longer sequences can be interspersed into the primer, provided that the primer sequence has sufficient complementarity with the sequence of the strand to hybridize therewith and thereby form the template for the synthesis of the extension product.

"Probes" refer to oligonucleotide nucleic acid sequences of variable length, used in the detection of identical, similar, or complementary nucleic acid sequences by hybridization. An oligonucleotide sequence used as a detection probe may be labeled with a detectable moiety". Various labeling moieties are known in the art. Said moiety may, for example, either be a radioactive compound, a detectable enzyme (e.g., horse radish peroxidase (HRP)) or any other moiety capable of generating a detectable signal such as a calorimetric, fluorescent, chemiluminescent or electrochemiluminescent signal. The detectable moiety may be detected using known methods.

As used herein, the term "protein" refers to a large molecule composed of one or more chains of amino acids in a specific order. The order is determined by the base sequence of nucleotides in the gene coding for the protein. Proteins are required for the structure, function, and regulation of the body's cells, tissues, and organs. Each protein has a unique function.

As used herein, the terms "quality traits," "traits," or "physical characteristics" refer to advantageous properties of the animal resulting from genetics. Quality traits include, but are not limited to, the animal's genetic ability to metabolize energy, produce milk, put on intramuscular fat, produce offspring, produce particular proteins in meat or milk, or retain protein in milk. Physical characteristics include, but are not limited to, marbled or lean meats, backfat, marbling, yield grade. The terms are used interchangeably.

A "restriction enzyme" refers to an endonuclease (an enzyme that cleaves phosphodiester bonds within a polynucleotide chain) that cleaves DNA in response to a recognition site on the DNA. The recognition site (restriction site) consists of a specific sequence of nucleotides typically about four to eight nucleotides long.

A "single nucleotide polymorphism" or "SNP" refers to polynucleotide that differs from another polynucleotide by a single nucleotide exchange. For example, without limitation, exchanging one A for one C, G, or T in the entire sequence of polynucleotide constitutes a SNP. Of course, it is possible to have more than one SNP in a particular polynucleotide. For example, at one locus in a polynucleotide, a C may be exchanged for a T, at another locus a G may be exchanged for an A, and so on. When referring to SNPs, the polynucleotide is most often DNA.

As used herein, a "template" refers to a target polynucleotide strand, for example, without limitation, an unmodified naturally-occurring DNA strand, which a polymerase uses as a means of recognizing which nucleotide it should next incorporate into a growing strand to polymerize the complement of the naturally-occurring strand. Such DNA strand may be single-stranded or it may be part of a double-stranded DNA template. In applications of the present invention requiring repeated cycles of polymerization, *e.g.*, the polymerase chain reaction (PCR), the template strand itself may become modified by incorporation of modified nucleotides, yet still serve as a template for a polymerase to synthesize additional polynucleotides.

A "thermocyclic reaction" is a multi-step reaction wherein at least two steps are accomplished by changing the temperature of the reaction.

A "thermostable polymerase" refers to a DNA or RNA polymerase enzyme that can withstand extremely high temperatures, such as those approaching 100°C. Often, thermostable polymerases are derived from organisms that live in extreme temperatures, such as *Thermus aquaticus.* Examples of thermostable polymerases include Taq, Tth, Pfu, Vent, deep vent, UlTma, and variations and derivatives thereof.

A "computer system" refers to the hardware means, software means and data storage means used to compile the data of the present invention. The minimum hardware means of computer-based systems of the invention may comprise a central processing unit (CPU), input means, output means, and data storage means. Desirably, a monitor is provided to visualize structure data. The data storage means may be RAM or other means for accessing computer readable media of the invention. Examples of such systems are, but not limited to, microcomputer workstations available from Silicon Graphics Incorporated and Sun Microsystems running Unix based Linux, Windows NT, IBM OS/2 operating systems and the like.

"Computer readable media" refers to any media which can be read and accessed directly by a computer, and includes, but is not limited to: magnetic storage media such as floppy discs, hard storage media and magnetic tape; optical storage media such as optical discs or CD-ROM; electrical storage media such as RAM and ROM; and hybrids of these categories, such as magnetic/optical media. By providing such computer readable media, the data compiled on a particular animal can be routinely accessed by a user, e.g., a feedlot operator.

The term "data analysis module" is defined herein to include any person or machine, individually or working together, which analyzes the sample and determines the genetic information contained therein. The term may include a person or machine within a laboratory setting or in a field setting such as in or near a feedlot or the like.

As used herein, the term "data collection module" refers to any person, object or system obtaining a tissue sample or determining the phenotype from an animal or embryo. By example, and without limitation, the term may define, individually or collectively, the person or machine in physical contact with the animal as the sample is taken, the containers holding the tissue samples, the packaging used for transporting the samples, and the like. Advantageously, the data collector is a person including, but not limited to, a livestock farmer, a breeder or a veterinarian.

The term "network interface" is defined herein to include any person or computer system capable of accessing data, depositing data, combining data, analyzing data, searching data, transmitting data or storing data. The term is broadly defined to be a person analyzing the data, the electronic hardware and software systems used in the analysis, the databases storing the data analysis, and any storage media capable of storing the data. Non-limiting examples of network interfaces include people, automated laboratory equipment, computers and computer networks, data storage devices such as, but not limited to, disks, hard drives or memory chips.

Unless otherwise defined, all technical and scientific terms used herein have the same meaning as commonly understood by one of ordinary skill in the art of molecular biology. Although methods and materials similar or equivalent to those described herein can be used in the practice or testing of the present invention, suitable methods and materials are described herein.

Further definitions are provided in context below. Unless otherwise defined, all technical and scientific terms used herein have the same meaning as commonly understood by one of ordinary skill in the art of molecular biology. Although methods and materials similar or equivalent to those described herein can be used in the practice or testing of the present invention, suitable methods and materials are described herein.

### Prediction of Revenue Yield per Marketing Method as Determined by Genotype Analysis

The present invention differs from current practice by using genetic test results to characterize animals. Rather than rely on a growth curve, ultrasound examination or visual inspection of animal traits, the present invention allows the farmer to feed livestock according to the individual animal's genetic traits and to select a marketing method that optimizes revenues from a group of animals. According to the method of the present invention, it is possible to select a desired trait, such as fat content, identify the gene which specifically encodes for a polypeptide associated with that trait, and genotype the animals possessing the associated gene.

The present invention further provides methods whereby the cattle producer or feedlot owner may determine the optimum marketing method, in particular a live weight, dressed weight or grid based estimation method that offers the highest revenue for a particular genotype. The determination may be performed before or after the animal enters the feedlot. This allows the feedlot owner to select individual cattle on the basis of their genotype that will predict with an increased reliability the expected carcass quality, especially with regard to meat quality at slaughter

In the example of fat content, it is known that leptin, a 16-kDa adipocyte-specific polypeptide, is encoded by the *ob* (obese) gene and appears to be involved in the regulation of appetite, basal metabolism and fat deposition. The *ob* gene has been mapped to specific chromosomes in several different animals, allowing the gene to be sequenced in several different species. Mutations in the coding sequences of the *ob* gene causing alterations in the amino acid sequence of the leptin polypeptide have been associated with hyperphagia, hypometabolic activity, and excessive fat deposition (a phenotype characterized by larger body size or fat phenotype). In the method of the present invention, it is possible to identify the absence or presence of a specific *ob* allele, thus predicting which animals may or may not possess certain carcass characteristics, e.g., increased fat deposition, increased mean fat deposition, increased percent rib fat, and decreased percent rib lean. For the ob gene, the presence of 138-bp allele was positively associated with these characteristics. Thus, bulls homogenous for the 138-bp allele exhibited greater average fat deposition than heterozygous animals.

The present invention provides methods wherein the genetic information obtained from individual animals is cross-matched against markers known in the art to be associated with specific characteristics. Keeping with the fat content example, it is known that a cytosine (C) to thymine (T) transition within an exon (exon 2) of the ob gene corresponds to an arginine (ARG) to cysteine (CYS) substitution in the leptin polypeptide. The exon 2 polymorphism is a C/T substitution located at position 305 of exon 2 of the bovine leptin gene (see, e.g., Buchanan et al. Genet Sel Evol. 34:105-16 (2002)). Thus, it is known that the presence of a T-containing allele in steers or heifers is predictive of fatter carcasses while bulls with a C-containing allele are known to be leaner.

The methods of the present invention comprise determining the genotypes of an animal or group of animals, most advantageously with regard to the SNP sites UASMS2 and EXON2-FB of the *ob* gene. The combination of these two SNPs provides reliable predictors of the carcass quality at time of slaughter. Thus, once the genotype of the animal is determined, each individual animal is evaluated as to whether it possesses the desired trait, i.e., possesses the specific gene. Animals having like genotypes for a specific gene/characteristic are then grouped together. These like-genotype groupings serve as the basis for breeding, feeding and determining slaughter time. Accordingly, the like-genotype groupings provide a more objective method for determining mates for breeding, diets and days on feed, and slaughter times.

The present invention provides systems and methods for determining the best marketing method of cattle by live weight, dressed weight, or grid basis, for different genotypes based upon the predictive ability of a genotype (haplotype) to forecast the final carcass quality after a period in the feedlot. The results from the simulation analyses using the methods of the invention indicate that the genotypes (E-CC;U-CC), (E-CC;U-CT), and (E-CT;U-CC) may best be marketed on a dressed weight basis, whereas (E-CC;U-TT) (E-CT;U-CT) (E-TT;U-CC), and "other" cattle genotypes may best be marketed on a live weight basis. Marketing a genotype via a sub-optimal method can generate significant economic losses. For example, as shown in Example 2 below, although (E-CC;U-CC) cattle might generate $910.19/head revenue when marketed on a dressed weight basis, they may only generate $904.67/head when marketed on a live weight basis, representing a loss of $5.52/head.

The systems and methods of the present invention further provide for analyses that allow the operator to determine which genotypes generate the highest revenue when all animals of a genotype are marketed via the same method. (E-CC;U-CC) cattle generate more revenue on average than the next best performing genotypes: "other" and (E-CT;U-CC). (E-CC;U-TT) cattle are the worst performing genotype.

The present invention further provides systems and methods to predict an animal's yield grade, quality grade, and dressing percentage when the genotypic and other background information are known, as shown, for example, in Table 6. These models form a tool to assist cattle producers in determining the types of cattle (especially which genotypes or mix of genotypes) to purchase and how to market them for slaughter. For example, when applied to a population of 1,668 cattle of mixed genotype for the markers UASMS2 and EXON, the results indicated that using the predictive models to market cattle by genotype could generate $2.76, $2.99, and $11.55/head more revenue than would naïvely marketing all of the animals as dressed weight, live weight, or grid, respectively. Therefore, while it is important to choose whether to market cattle on a live weight, dressed weight, or grid basis, it is more important to start with the optimal kind of cattle. The systems and methods of the invention, therefore, are also useful in allowing cattle producers to purchase and feed animals of certain genotypes, while avoiding cattle of other genotypes, thereby maximizing the revenue from a population of cattle.

The methods according to the present invention provide estimated prediction equations useful in determining an animal's expected yield grade, marbling score, and dressing percentage to determine the value of using genetic information to improve marketing decisions. Exemplary prediction models for several carcass traits are shown in Examples 7-12 below. Based on these predicted values, the expected revenue from each marketing method (live weight, dressed weight and grid) may be calculated. Each animal may then be targeted to the marketing method that offers the highest *expected* revenue given the expected carcass characteristics. The following steps are used to calculate revenue from this method: (a) determine live weight price, dressed weight price, and grid premiums and discounts according to predetermined statistical data; (b) predict the yield grade, quality grade, and dressing percentage using the estimated prediction equations; (c) given the carcass predictions and predefined market prices, calculate the expected revenue for live weight, dressed weight, and grid marketing methods; (d) market each animal by the method that produces the greatest expected revenue; and (e) calculate the actual revenue, using actual carcass characteristics and predefined prices, when animals are marketed in the marketing manner described by step (d).

### *Methods of Grouping and Selecting Animals According to SNP Genotype*

The present invention provides methods for the identification and selection of animals based on the presence of SNPs in the *ob* (obese) gene - a gene that encodes the protein leptin- and computer-based systems and methods for predicting optimal marketing of cattle based upon their genotype with regard to the *ob* gene.

In the present invention it has been shown that in bovine livestock, the genotype of the animal with respect to a pair of SNPS, namely UASMS2 and EXON2-FB is associated with a number of carcass traits of economic interest, in particular, but not limited to, hot carcass weight (HCW), ribeye area (REA), dressing percentage (DP), backfat (BF), yield grade (YG), marbling score (MBS) and marbling score per days of feeding (MBS/DOF).

The SNP termed "UASMS2" constitutes a cytosine (C) to thymine (T) substitution (C/T substitution) at position 528 of bovine leptin gene promoter. The SNP termed EXON2-FB was identified previously by Buchanan et al. (2002), and constitutes a cytosine (C) to thymine (T) missense mutation at position 1759 in exon 2 of the coding region of the "wild type" bovine leptin gene (GenBank accession No. AY138588). The nucleotide numbering system used herein for the identification of the EXON2-FB SNP is that used for the "wild type" bovine leptin exon 2 sequence. These and other SNPs associated with the bovine ob gene useful in the practice of the present invention, their isolation and sequence characterizations are described in detail in, *inter alia,* U.S. Patent Application Serial No: 10/891,256 incorporated herein by reference in its entirety. Methods for the obtaining of biological samples from subject cattle and the determination of their genotypes using probes specific for the SNPs of the ob gene, especially of UASMS2 and EXON2-FB are also described in detail in, *inter alia,* US Patent Application Serial No: 11/061,942 incorporated herein by reference in its entirety.

In addition to the SNPs described above, it will be appreciated by those skilled in the art that other DNA sequence polymorphisms of the *ob* gene may exist within a population. Such natural allelic variations can typically result in about 1 to 5% variance in the nucleotide sequence of the gene. It is possible that other polymorphic loci may also exist within this fragment. In addition to naturally-occurring allelic variants of the nucleotide sequence, the skilled artisan will further appreciate that changes can be introduced by mutation into the nucleotide sequence of the nucleotide sequences described herein. Any and all such additional nucleotide variations are intended to be within the scope of the invention.

Thus, for example, a probe according to the present invention may be designed to bind to a sequence of the *ob* gene containing not only the UASMS2 polymorphism, but also other SNPs that may occur within the same region.

Genetic tendencies can be predicted by the results of genotyping. A method and system of the invention comprises tissue sampling, extraction of genetic material from the sampled tissue, molecular genetic analysis of the genetic material, and where the tissue sample is taken from a meat product, comparison of the genotype with known animal genotypes stored on a database. It is contemplated by the methods and systems described herein that the continuity and integrity of each sample is maintained so that the data is accurate and reliable. Steps necessary for ensuring that the data is accurate and reliable are included in the methods and systems taught herein.

Additionally, the method of the present invention contemplates grouping animals according to their genotype in addition to using the phenotype criteria currently employed in feeding, breeding or growing practices. For example, in one embodiment of the present invention, feedlot operators who currently group livestock according to size and frame, among other phenotypic traits, would use the data obtained from animals' genotypes which correspond to an animal's propensity to exhibit a characteristic associated with the particular gene, and optionally any other associated data, in order to more efficiently manage production. Thus, the feeder is presented with opportunities for considerable efficiencies in livestock production.

Presently, the feeder feeds all his cattle the same, incurring the same costs for each animal, and typically, with excellent management practices, perhaps 40% will receive an optimal grade of Choice or Prime, and receive the premium price for the quality grade. Of these, a significant number will have excess fat and will thus receive a reduced yield grade. The balance of the cattle, 60%, will grade less than Choice or Prime, and thus receive a reduced price, although the feedlot costs incurred by the feeder are substantially the same for these cattle receiving the lesser grade. Grouping and feeding cattle by genotype allows the feeder to treat each group differently with a view to optimizing management strategies and increasing profits or, if necessary, determining the days on feed for a mixed group of cattle that will provide the highest revenue possible.

Using the methods as described in U.S. Patent Application Serial No: 11/061,942, and incorporated herein by reference in its entirety, one can determine whether a given animal has a cytosine or a thymine at the polymorphic UASMS1 locus (located at nucleotide position 207 of the *ob* gene promoter), a cytosine or a thymine at the polymorphic UASMS2 (located at nucleotide position 528 of the *ob* gene promoter), a cytosine or a guanine at the polymorphic UASMS3 (located at nucleotide position 1759 of the *ob* gene promoter) and a cytosine or a thymine at the polymorphic EXON2-FB (located at nucleotide position 1180 in leptin gene). Having used the methods of the invention to determine the genotype of an animal of interest, for example, at the UASMS2 and EXON2-FB polymorphic loci, it is a further object of the present invention to utilize this genotype information to select and/or group animals according to their genotype.

Certain alleles of the UASMS1, UASMS2, UASMS3 and EXON2-FB SNPs are associated with certain economically important traits such as circulating leptin levels, feed intake, growth rate, body weight, carcass merit and composition, and milk yield. For example, the T allele of the UASMS2 locus is associated with serum leptin concentration, being lowest in homozygous animals with the CC genotype, intermediate in heterozygous animals with the CT genotype, and highest in homozygous TT animals. Thus, in one embodiment, where it is desirable to group animals according to circulating leptin concentration (for example for use in food production or for breeding), animals can be selected and grouped according to their genotype at the polymorphic UASMS2 locus. Associations between the genotypes of each of the UASMS2 and EXON2-FB polymorphic loci and combinations thereof, and various other economically important traits, are described in the Examples. Thus, for each of these traits, animals can be grouped according to genotype.

Thus, in one embodiment, the present invention provides methods for grouping animals and methods for managing livestock production comprising grouping livestock animals, such as cattle according to genotype of, but not limited to, the SNP pair UASMS2 and EXON2-FB polymorphic loci. It is contemplated that the combination of UASMS2 and EXON2-FB is especially advantageous in the practice of the present invention. The genetic selection and grouping methods of the present invention can be used in conjunction with other conventional phenotypical grouping methods such as grouping animals by characteristics such as weight, frame size, breed traits, and the like.

The methods of the present invention provide for selecting cattle having improved heritable traits, and can be used to optimize the performance of livestock herds in areas such as breeding, feed consumption, carcass/meat quality and the like. The present invention provides methods of screening livestock to determine those more likely to develop a desired body condition by identifying the presence or absence of polymorphism in the *ob* gene that are correlated with that body condition, especially genotypes of the UASMS2-EXON2-FB pair.

As described above, and in the Examples below, there are various phenotypic traits with which the SNPs of the present invention are associated. Each of the phenotypic traits can be tested using the methods as described in U.S. Patent Application Serial No: 11/061,942, herein incorporated by reference in its entirety, or using any suitable methods known in the art. Using the methods of the invention, a farmer, feedlot operator or the like can group cattle according to each animal's genetic propensity for a desired trait such as circulating leptin levels, feed intake, growth rate, body weight, carcass merit and composition, backfat, ribeye area, marbling, yield grade and the like, as determined by SNP genotype, in addition to the present criteria he would ordinarily use for grouping. The cattle may be tested to determine homozygosity or heterozygosity with respect to UASMS2 and EXON2-FB alleles of the ob gene so that they can be grouped such that each pen contains cattle with like genotypes.

Each pen of animals may then be fed and otherwise maintained in a manner and for a time determined by the feedlot operator to be ideal for meat production prior to slaughter. Thus the farmer or feedlot operator is presented with opportunities for considerable efficiencies. At present, the feeder feeds all his cattle the same, incurring the same costs for each animal, and typically, with excellent management practices, perhaps 40% will grade choice and receive the premium price for the palatability grade. Grouping and feeding the cattle by genotype allows the farmer to treat each group differently with a view to increasing profit.

It is contemplated that, regardless of the desirability and premium paid for any particular meat quality at any given time, providing a more uniform group of animals that have a predictable meat quality will provide the rancher, feeder, or feedlot operator with the opportunity to demand and receive a premium, relative to the less uniform groups of cattle presently available.

A sampling device and/or container may be supplied to take a consistent and reproducible sample from individual animals while simultaneously avoiding any cross-contamination of tissue. Accordingly, the size and volume of sample tissues derived from individual animals would be consistent.

A tissue sample may be taken from an animal at any time in the lifetime of an animal but before the carcass identity is lost. The tissue sample can comprise hair, including roots, hide, bone, buccal or nasal swabs, blood, saliva, milk, semen, embryos, muscle or any internal organs.

The tissue sample is marked with an identifying number or other indicia that relates the sample to the individual animal from which the sample was taken. The identity of the sample advantageously remains constant throughout the methods and systems of the invention thereby guaranteeing the integrity and continuity of the sample during extraction and analysis. Alternatively, the indicia may be changed in a regular fashion that ensures that the data, and any other associated data, can be related back to the animal from which the data was obtained.

The amount/size of sample required is known to those skilled in the art and for example, can be determined by the subsequent steps used in the method and system of the invention and the specific methods of analysis used. Ideally, the size/volume of the tissue sample retrieved should be as consistent as possible within the type of sample and the species of animal. For example, for cattle, non-limiting examples of sample sizes/methods include non-fatty meat: 0.0002 gm to about 0.0010 gm; hide: 0.0004 gm to about 0.0010 gm; hair roots: at least five and advantageously between about twenty to about thirty; buccal swabs: 15 to 20 seconds of rubbing with modest pressure in the area between outer lip and gum using one Cytosoft® cytology brush; bone: 0.0020 gm to about 0.0040 gm; blood: about 30 $\mu$l to about 70$\mu$l.

Generally, the tissue sample is placed in a container that is labeled using a numbering system bearing a code corresponding to the animal, for example, to the animal's ear tag. Accordingly, the genotype of a particular animal is easily traceable at all times.

The tissue sample is then treated by the desired methods to retrieve the desired data, for example, the genotype. Alternatively, the samples can be frozen for preservation and archived, for example, in the factory/slaughterhouse or a central storage location for future extraction/analysis as required.

In the present invention, a sample of genomic DNA is obtained from an animal. For example, peripheral blood cells may be used as the source of the DNA. A sufficient amount of cells are obtained to provide a sufficient amount of DNA for analysis. This amount will be known or readily determinable by those skilled in the art. The DNA is isolated from the blood cells by techniques known to those skilled in the art (see, e.g., U.S. Patent Nos. 6,548,256 and 5,989,431, Hirota et al., Jinrui Idengaku Zasshi. 34: 217-23 (1989) and John et al., Nucleic Acids Res. 19: 408 (1991); the disclosures of which are incorporated by reference in their entireties).

In the method of the present invention, the source of the test nucleic acid is not critical. For example, the test nucleic acid can be obtained from cells within a body fluid of the livestock or from cells constituting a body tissue of the subject. The particular body fluid from which the cells are obtained is also not critical to the present invention. For example, the body fluid may be selected from the group consisting of blood, ascites, pleural fluid and spinal fluid. Furthermore, the particular body tissue from which cells are obtained is also not critical to the present invention. For example, the body tissue may be selected from the group consisting of skin, endometrial, uterine and cervical tissue. Both normal and tumor tissues can be used. Further, the source of the target material may include RNA or mitochondrial DNA.

The invention further comprises methods of screening livestock, advantageously cattle, to determine those having predictably more uniform fat deposition based upon the presence or absence of certain polymorphisms in the ob gene. In one embodiment, the ob gene polymorphism is a C to T transition that results in an Arg25Cys in the leptin protein. One of ordinary skill in the art can apply the methods described herein for detecting polymorphisms of the ob gene to detect any other genotypes or polymorphisms correlating to a particular phenotype. Indications of high densities of SNPs in defined regions in the bovine subspecies Bos taurus and Bos indices have been found (reviewed in Vignal et al., Genet. Sel. Evol. 34: 275-305(2002), the disclosure of which is incorporated by reference in its entirety).

Suitable probes and primers for the detection of polymorphism at the polymorphic sites of the cow genome are described in detail in U.S. Patent Application Serial No. 11/061,942, herein incorporated by reference in its entirety. Primers may be of any length but, typically, are about 10 to about 24 bases in length. A probe or primer can be any stretch of at least 8, advantageously at least 10, more advantageously at least 12, 13, 14, or 15, such as at least 20, e.g., at least 23 or 25, for instance at least 27 or 30 nucleotides. As to PCR or hybridization primers or probes and optimal lengths therefore, reference is also made to Kajimura et al., GATA 7(4):71-79 (1990), the disclosure of which is incorporated by reference in its entirety. In certain embodiments, it is contemplated that multiple probes may be used for hybridization to a single sample. Designing and testing the probes and primers around the *ob* nucleotide sequences described above and from any one of the sequences corresponding to the accession numbers listed can be accomplished by one of ordinary skill in the art.

In addition to leptin, the invention may encompass genetic testing of other genes and SNPs that correlate to a particular phenotype. One of ordinary skill in the art can easily apply the exemplified techniques described herein for leptin to other SNPs, genotypes and polymorphisms that correlate with a particular phenotype, physical characteristic or trait. For example, the design of an oligonucleotide primer to amplify a sequence (e.g., containing a genetic polymorphism of interest) of a given gene is routine experimentation for one of ordinary skill in the art. Such genes and/or SNPs include, but are not limited to, BGHR, calpain, calpastatin, CXCR2, DGAT1, FAA, TIMP2, IGF-1, IGF-2, POMC, neuropeptide Y, leptin receptor, thyroglobulin, UCP2 and UCP3.

The present invention incorporates a method of detecting the presence of ob gene polymorphisms in a specimen wherein the oligonucleotides of the present invention may be used to amplify target nucleic acid sequences of an *ob* gene polymorphism that may be contained within a livestock specimen, and/or to detect the presence or absence of amplified target nucleic acid sequences of the *ob* gene polymorphism. Respective oligonucleotides may be used to amplify and/or detect *ob* gene and *ob* gene nucleic acid sequences. As few as one to ten copies of the *ob* gene polymorphism may be detected in the presence of milligram quantities of extraneous DNA.

Methods are provided for the analysis and determination of SNPs in a genetic target. In this embodiment, both wild type and mutant alleles are distinguished, if present in a sample, at a single capture site by detecting the presence of hybridized allele-specific probes labeled with fluorophores sensitive to excitation at various wave lengths.

A target nucleic acid can be first amplified, such as by PCR or SDA. The amplified dsDNA product is then denatured and hybridized with a probe. The hybridization complex formed is then subjected to destabilizing conditions to differentiate and identify the *ob* SNP.

Methods of detecting the presence of an *ob* gene polymorphism in a sample include, but are not limited to, contacting the sample with the above-described nucleic acid probe, under conditions such that hybridization occurs, enzymatically amplifying a specific region of the *ob* gene nucleic acid molecules, and c) detecting the presence of the probe bound to the DNA segment.

Any one of the methods commercially available may accomplish amplification of DNA. For example, the polymerase chain reaction may be used to amplify the DNA. Once the primers have hybridized to opposite strands of the target DNA, the temperature is raised to permit replication of the specific segment of DNA across the region between the two primers by a thermostable DNA polymerase. Then the reaction is thermocycled so that at each cycle the amount of DNA representing the sequences between the two primers is doubled, and specific amplification of the *ob* gene DNA sequences, if present, results.

Further identification of the amplified DNA fragment, as being derived from *ob* gene DNA, may be accomplished by liquid hybridization. This method utilizes one or more oligonucleotides labeled with detectable moiety as probes to specifically hybridize to the amplified segment of *ob* gene DNA. Detection of the presence of sequence-specific amplified ob gene DNA may be accomplished by simultaneous detection of the complex comprising the labeled oligonucleotide hybridized to the sequence-specific amplified ob gene DNA ("amplified target sequences") with respect to the DNA amplification. Detection of the presence of sequence-specific amplified *ob* gene DNA may also be accomplished using a gel retardation assay with subsequent detection of the complex comprising the labeled oligonucleotide hybridized to the sequence-specific amplified *ob* gene DNA.

The use of a hybridization probe of between 10 and 30 nucleotides in length allows the formation of a duplex molecule that is both stable and selective. Molecules having complementary sequences over stretches greater than 12 bases in length are generally advantageous, in order to increase stability and selectivity of the hybrid, and thereby improve the quality and degree of particular hybrid molecules obtained. One will generally prefer to design nucleic acid molecules having stretches of 16 to 24 nucleotides, or even longer where desired. Such fragments may be readily prepared by, for example, directly synthesizing the fragment by chemical means or by introducing selected sequences into recombinant vectors for recombinant production.

In such an enzymatic amplification reaction hybridization system of *ob* gene allele detection, a specimen of blood, CSF, amniotic fluid, urine, body secretions, or other body fluid is subjected to a DNA extraction procedure. High molecular weight DNA may be purified from blood cells, tissue cells or virus particles (collectively referred to herein as "cells") contained in the livestock specimen using proteinase (proteinase K) extraction and ethanol precipitation. DNA may be extracted from a livestock specimen using other methods known in the art. Then, for example, the DNA extracted from the livestock specimen is enzymatically amplified in the polymerase chain reaction using *ob* gene-specific oligonucleotides as primer pairs. Following amplification, *ob* gene-specific oligonucleotides labeled with an appropriate detectable label are hybridized to the amplified target sequences, if present.

The contents of the hybridization reaction are then analyzed for detection of the sequence-specific amplified *ob* gene DNA, if present in the DNA extracted from the livestock specimen. Thus, the oligonucleotides of the present invention have commercial applications in diagnostic kits for the detection of *ob* gene DNA in livestock specimens.

The test samples suitable for nucleic acid probing methods of the present invention include, for example, cells or nucleic acid extracts of cells, or biological fluids. The sample used in the above-described methods will vary based on the assay format, the detection method and the nature of the tissues, cells or extracts to be assayed. Methods for preparing nucleic acid extracts of cells are well known in the art and can be readily adapted in order to obtain a sample that is compatible with the method utilized.

The results of the analysis provide the genotype data that is associated with the individual animal from which the sample was taken. The genotype data is then kept in an accessible database, and may or may not be associated with other data from that particular individual or from other animals.

### Computer Based Systems for Prediction of Optimal Marketing Method as Determined from Cattle Genotype

The data obtained from genotyping individual animals is stored in a database which can be integrated or associated with and/or cross-matched to other databases. The database along with the associated data allows information about the individual animal to be known through every stage of the animal's life, i.e., from conception to consumption of the animal product.

The accumulated data, the combination of the genetic data with other types of data of the animal provides access to information about parentage, identification of herd, health information including vaccinations, exposure to diseases, feedlot location, diet and ownership changes. Information such as dates and results of diagnostic or routine tests are easily stored and attainable. Such information would be especially valuable to companies specializing in artificial insemination of animals, particularly those who seek superior breeding lines.

Each animal is provided with a unique identifier. The animal can be tagged, as in traditional tracing programs or have implant computer chips providing stored and readable data or provided with any other identification method which associates the animal with its unique identifier.

The database containing the genotype results for each animal or the data for each animal can be associated or linked to other databases containing data, for example, which may be helpful in selecting traits for grouping or sub-grouping of an animal. For example, and not for limitation, data pertaining to animals grouped for propensity to lay fat can be linked with data pertaining to animals having particular hormone levels, and optionally can be further linked with data pertaining to animals having feed from a particular feed source. The ability to refine a group of animals is limited only by the traits sought and the databases containing information related to those traits.

Databases with which the genotyping data can be associated include specific or general scientific data. Specific data includes, but is not limited to, breeding lines, sires, and the like, other animals' genotypes, including whether or not other specific animals possess specific genes, location of animals which share similar or identical genetic characteristics, and the like. General data includes scientific data such as which genes encode for specific quality characteristics, breed association data, feed data, breeding trends, and the like.

A method of the present invention may also include providing the animal owner or customer with sample collection equipment, such as swabs and vials. The collection equipment may be packaged in a container which is encoded with identifying indicia. Advantageously, the packaging is encoded with a bar code label. The collection equipment is encoded with the same identifying indicia, advantageously with a matching bar code label. Optionally, the packaging contains means for sending the collection equipment to a laboratory for analysis. The optional packaging is also encoded with identifying indicia, advantageously with a bar code label.

The method optionally includes a system wherein a database account is established upon ordering the sampling equipment. The database account identifier corresponds to the identifying indicia of the collection equipment and the packaging. Upon shipment of the sampling equipment in fulfillment of the order, the identifying indicia are recorded in a database. Advantageously, the identifier is a bar code label which is scanned when the vials are sent. When the vials are returned to the testing facility, the identifier is again recorded and matched to the information previously recorded in the database upon shipment of the vial to the customer. Once the genotyping is completed, the information is recorded in the database and coded with the unique identifier. Test results are also provided to the customer or animal owner.

The database is accessible to those to whom access has been provided. Access can be provided through rights to access or by subscription to specific portions of the data. For example, the database can be accessed by owners of the animal, the test site, the entity providing the sample to the test site, feedlot personnel, and veterinarians. The data can be provided in any form such as by accessing a website, fax, email, mailed correspondence, automated telephone, or other methods for communication. This data can also be encoded on a portable storage device, such as a microchip, that can be implanted in the animal. Advantageously, information can be read and new information added without removing the microchip from the animal.

The present invention comprises systems for performing the methods disclosed herein. Such systems comprise devices, such as computers, internet connections, servers, and storage devices for data. The present invention also provides for a method of transmitting data comprising transmission of information from such methods herein discussed or steps thereof, e.g., via telecommunication, telephone, video conference, mass communication, e.g., presentation such as a computer presentation, internet, email, documentary communication such as a computer program-generated document and the like.

Systems of the present invention comprise a data collection module, which includes a data collector to collect data from an animal or embryo and transmit the data to a data analysis module, a network interface for receiving data from the data analysis module, and optionally further adapted to combine multiple data from one or more individual animals, and to transmit the data via a network to other sites, or to a storage device.

More particularly, systems of the present invention comprise a data collection module, a data analysis module, a network interface for receiving data from the data analysis module, and optionally further adapted to combine multiple data from one or more individual animals, and to transmit the data via a network to other sites, and/or a storage device. For example, the data collected by the data collection module leads to a determination of the absence or presence of an allele of the *ob* gene in the animal or embryo, and for example, such data is transmitted to a feedlot when the feeding regimen of the animal is planned.

The invention also provides for accessing other databases, e.g., herd data relating to genetic tests and data performed by others, by datalinks to other sites. Therefore, data from other databases can be transmitted to the central database of the present invention via a network interface for receiving data from the data analysis module of the other databases.

A computer readable media may contain such data as described above. The invention also relates to a method of doing business comprising providing to a user the computer system described herein or the media described herein.

The invention provides for a computer-assisted method for predicting which livestock animals possess a physical characteristic comprising: using a computer system, e.g., a programmed computer comprising a processor, a data storage system, an input device and an output device, the steps of: (a) inputting into the programmed computer through the input device comprising a genotype of an animal, (b) correlating a physical characteristic predicted by the genotype using the processor and the data storage system and (c) outputting to the output device the physical characteristic correlated to the genotype, thereby predicting which livestock animals possess a physical characteristic.

The invention also provides for a computer-assisted method for improving livestock production comprising: using a computer system, e.g., a programmed computer comprising a processor, a data storage system, an input device and an output device, the steps of: (a) inputting into the programmed computer through the input device comprising a genotype of an animal, (b) correlating a physical characteristic predicted by the genotype using the processor and the data storage system, (c) outputting to the output device the physical characteristic correlated to the genotype and (d) feeding the animal a diet based upon the physical characteristic, thereby improving livestock production.

The invention further provides for a computer-assisted method for optimizing efficiency of feedlots for livestock comprising: using a computer system, e.g., a programmed computer comprising a processor, a data storage system, an input device and an output device, the steps of: (a) inputting into the programmed computer through the input device comprising a genotype of an animal, (b) correlating a physical characteristic predicted by the genotype using the processor and the data storage system, (c) outputting to the output device the physical characteristic correlated to the genotype and (d) feeding the animal a diet based upon the physical characteristic, thereby optimizing efficiency of feedlots for livestock.

In one advantageous embodiment, the genotype is an ob genotype. In this embodiment, the physical characteristic correlating to a CC genotype is a low propensity to deposit fat, the physical characteristic correlating to a TT genotype is a high propensity to deposit fat and the physical characteristic correlating to a CT genotype is an intermediate propensity to deposit fat.

Accordingly, one aspect of the invention is a computer-assisted method for determining revenue from a cattle marketing method comprising using a programmed computer comprising a processor, a data storage system, an input device and an output device, and the steps of: (a) determining the genotype of an animal or group of animals by identifying at least two single length polymorphism of the animal or animals; (b) inputting data into the programmed computer through the input device, wherein the data comprises a genotype of an animal, a physical characteristic of the animal at placement, a carcass prediction and a plurality of predefined market prices; (c) calculating a revenue expectation from a cattle marketing method for a plurality of genotypes by calculating a live weight price, a dressed weight price and a grid price for each genotype, wherein the grid price comprises premium and discount prices; (d) correlating the expected revenues with the genotypes and the marketing methods; and (e) outputting to the output device the expected revenues for the cattle marketing methods.

In one embodiment of this aspect of the invention, the method further comprises the step of identifying the marketing method providing the highest revenue for an animal or group of animals.

In another embodiment of this aspect of the invention, the method further comprises the step of identifying the marketing method providing the highest revenue for a genotype.

In the various embodiments of this aspect of the invention, the input data may be selected from a genotype, placement weight, ultrasound backfat measurement at placement, frame score at placement, days on feed, and gender.

In other embodiments of the invention, the method may further comprise the steps of (a) calculating the predicted values of yield grade, quality grade, and dressing percentage of an animal for the cattle marketing method by using at least one estimated prediction equation defining the change in a trait of an animal over a period of feeding; (b) calculating the expected revenues for live weight, dressed weight and grid marketing methods by using the predicted values of yield grade, the quality grade, and the dressing percentage; (c) determining the marketing method giving the highest expected revenue for the animal.

In various embodiments of the present invention, a trait of an animal predicted by an equation is selected from the group of traits consisting of backfat production, marbling score, weight, dressing percentage, dry matter intake and ribeye area.

In one embodiment of the invention, the method of calculating the predicted values of yield grade, quality grade, and dressing percentage of an animal for the cattle marketing is by using a plurality of prediction equations, wherein the predictive equations determines the changes in a plurality of traits of an animal over a period of feeding.

In various embodiments of the invention, the genotype of the animal is determined from at least two single-length polymorphisms of the *ob* gene, thereby defining a genotype of the subject animal(s). In one embodiment of the invention, the single-length polymorphisms are UASMS2 and EXON2-FB of the *ob* gene.

In yet another embodiment of the invention, the method further comprises the step of marketing each animal by the marketing method that produces the greatest expected revenue.

In the embodiments of the invention, the genotype may be an ob genotype, wherein a physical characteristic correlating to a CC genotype is a low propensity to deposit fat, the physical characteristic correlating to a TT genotype is a high propensity to deposit fat and the physical characteristic correlating to a CT genotype is an intermediate propensity to deposit fat. It is also intended that other correlations with the ob genotypes may be useful in the methods of the present invention, including but not limited to, marbling score, weight, dressing percentage, dry matter intake and ribeye area.

The invention further encompasses embodiments, wherein the methods further comprise the step of transmitting data comprising transmission of information from the methods via telecommunication, telephone, video conference, mass communication, presentation graphics, internet, email, or paper or electronic documentary communication.

A computer-assisted method for determining revenue from a cattle marketing method comprising using a programmed computer comprising a processor, a data storage system, an input device and an output device, and the steps of: (a) determining the genotype of an animal or group of animals by identifying the genotype of the animal or animals, wherein single-length polymorphisms defining the genotype are UASMS2 and EXON2-FB of the *ob* gene; (b) inputting data into the programmed computer through the input device, wherein the data comprises a genotype of an animal and at least one physical characteristic of the animal at placement, a carcass prediction and a plurality of predefined market prices, genotype, placement weight, ultrasound backfat measurement at placement, frame score at placement, days on feed, and gender; (c) calculating a revenue expectation from a cattle marketing method for a plurality of genotypes by calculating a live weight price, a dressed weight price, and a grid price for each genotype, wherein the grid price comprises premium and discount prices; (d) correlating the expected revenues with the genotypes and the marketing methods; (e) calculating the predicted values of the yield grade, the quality grade, and the dressing percentage of an animal for the cattle marketing method by using a plurality of estimated prediction equations, wherein the predictive equations determine the changes in a plurality of traits of an animal over a period of feeding, and wherein the traits of an animal is selected from the group of traits consisting of backfat production, marbling score, weight, dressing percentage, dry matter intake and ribeye area; (f) calculating the expected revenues for live weight, dressed weight and grid marketing methods by using the predicted values of yield grade, quality grade, and dressing percentage; (g) determining the marketing method giving the highest predicted expected revenue for the animal; (h) identifying the marketing method providing the highest revenue for an animal or group of animals; (i) outputting to the output device the expected revenues for the cattle marketing methods; and (j) marketing each animal by the marketing method that produces the greatest expected revenue.

### Kits for the Determination of the Genotype of an Animal or Group or Animals

Oligonucleotide primers and probes useful in the practice of the present invention and as described in full in U.S. Patent Application Serial No. 11/061,942, herein incorporated by reference in its entirety, have commercial applications in diagnostic kits for the detection of the UASMS1, UASMS2, UASMS3 and EXON2-FB ob gene SNPs in livestock specimens. A test kit according to the invention may comprise any of the oligonucleotide primers or probes according to the invention. Such a test kit may additionally comprise one or more reagents for use in cyclic polymerase mediated amplification reactions, such as DNA polymerases, nucleotides (dNTPs), buffers, and the like. An SNP detection kit may also include, a lysing buffer for lysing cells contained in the specimen.

A test kit according to the invention may comprise at least two pairs of oligonucleotide primers according to the invention and a probe capable of detecting the amplified regions comprising at least one oligonucleotide as described in full in U.S. Patent Application Serial No. 11/061,942, herein incorporated by reference in its entirety. In some embodiments such a kit will contain two allele specific oligonucleotide probes. Advantageously, the kit further comprises additional means, such as reagents, for detecting or measuring the binding or the primers and probes of the present invention, and also ideally a positive and negative control.

Unless otherwise defined, all technical and scientific terms used herein have the same meaning as commonly understood by one of ordinary skill in the art of molecular biology, molecular genetics, animal science, animal husbandry and the like. Although methods and materials similar or equivalent to those described herein can be used in the practice or testing of the present invention, suitable methods and materials are described herein. All publications, patent applications, patents, and other references mentioned herein are incorporated by reference in their entirety. In addition, the materials, methods, and examples are illustrative only and are not intended to be limiting.

## EXAMPLES

### Example 1

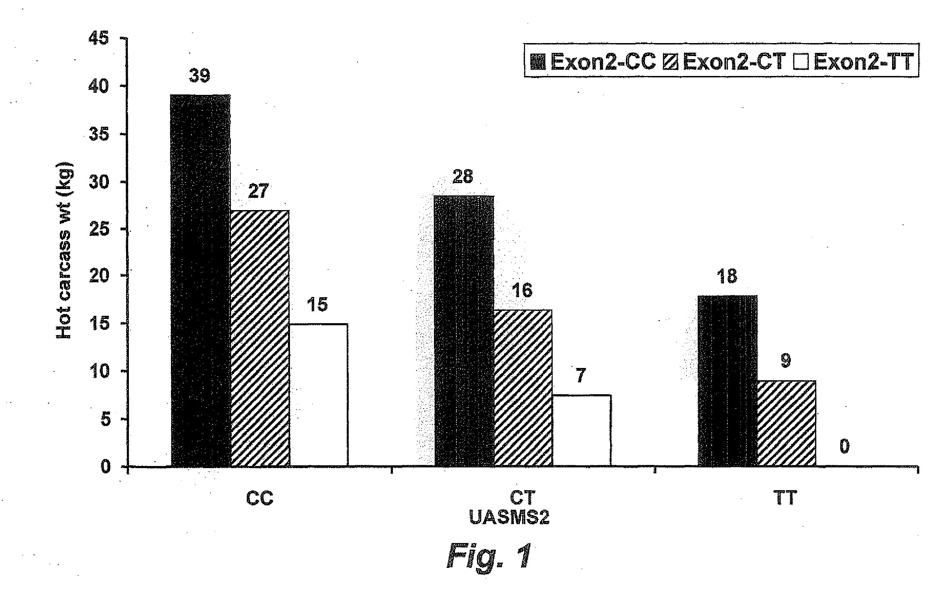

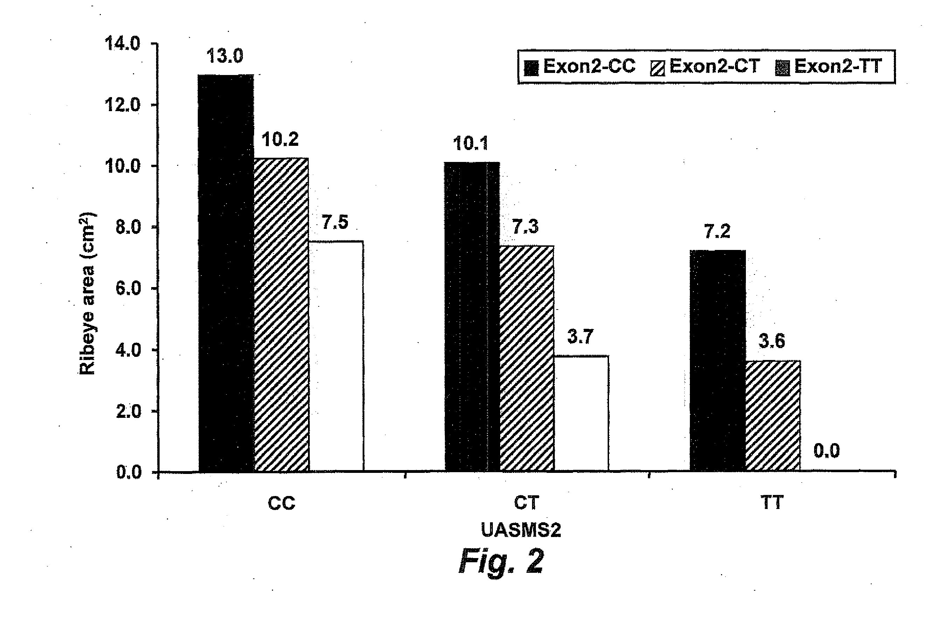

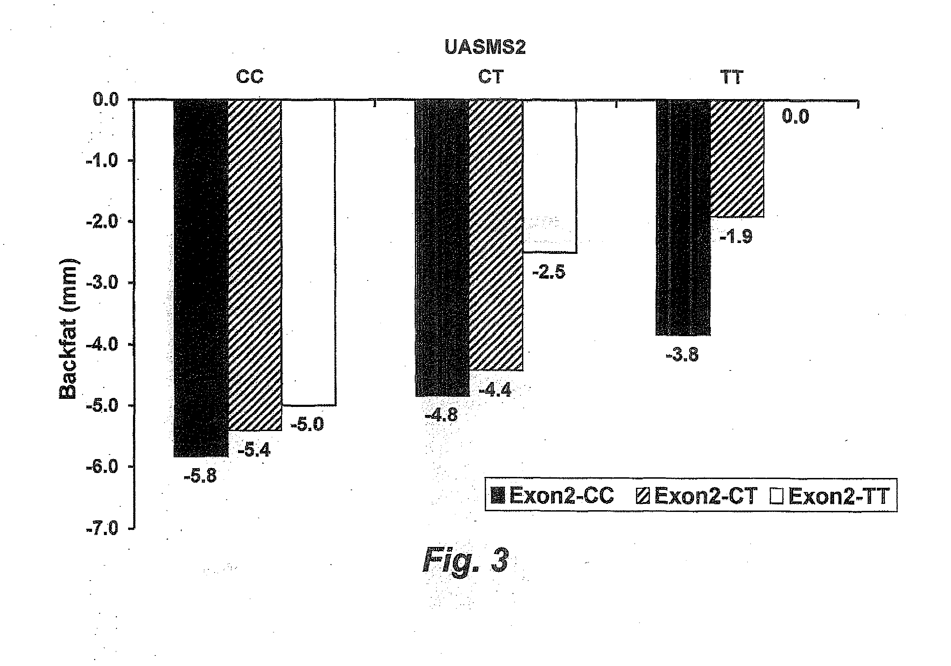

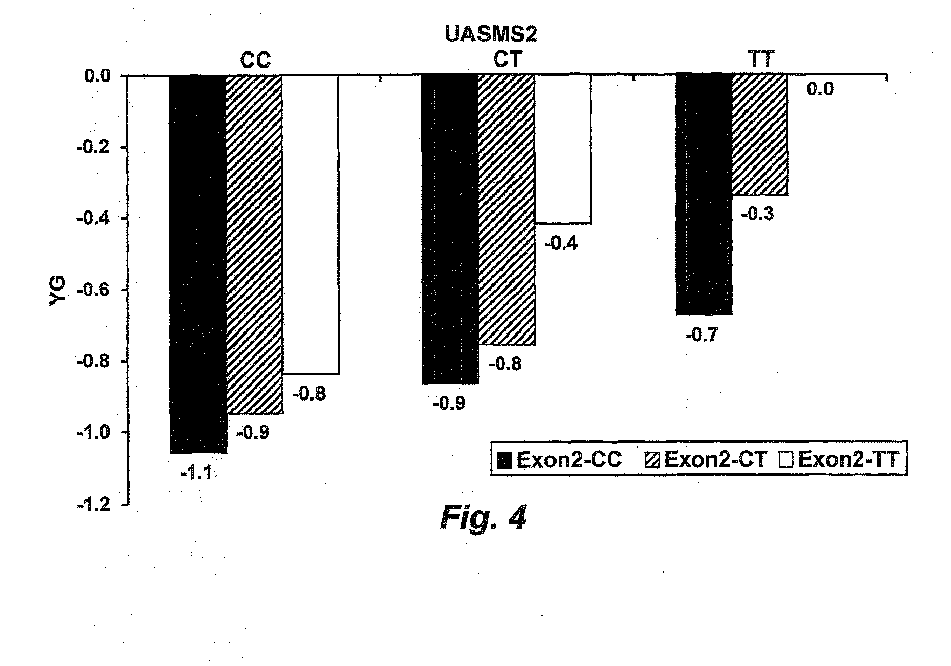

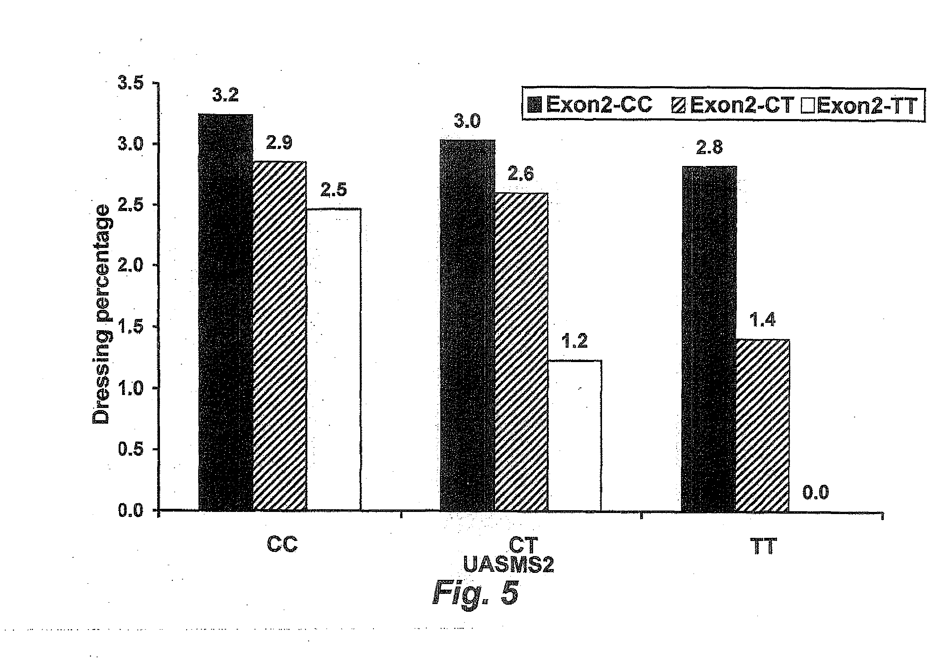

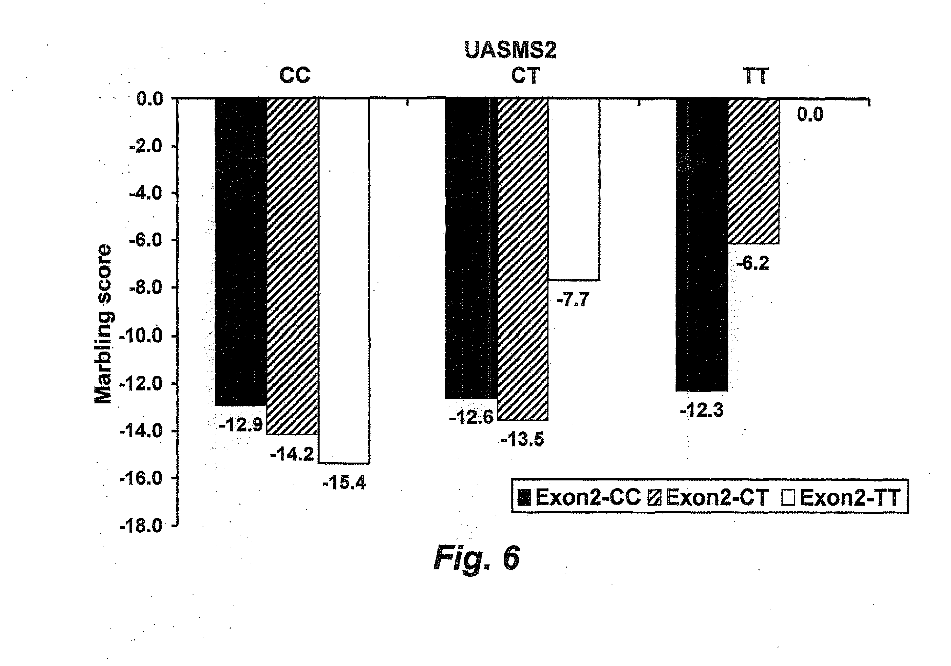

The correlations between a particular genotype for the ob gene locus with respect to the markers UASMS2 and EXON2-FB for one group of cattle are illustrated in Figs 1-6. For example, the greatest hot carcass weight is found for animals having the genotype UASMS2 CC; EXON2-FB CC (Fig 1), whereas a reduction in marbling score is seen in UASMS2 TT; EXON2-FB TT animals compared to UASMS2 CC; EXON2-FB CC cattle (Fig. 6).

The experimental data set contained individual-animal information for another group with 2,172 head of cattle. Since complete information was not available on all animals, the results were finally based on 1,668 head of cattle from this group. Although initial genetic information was provided for the genetic markers UASMS1, UASMS2, EXON2-FB, GHR, and DGAT, it was determined that a combination of the UASMS2 (referred to hereafter by the acronym U) and EXON2-FB (referred to hereafter by the acronym E) markers provided the most useful information. Data sets, therefore, focused on seven genotypes (E-CC;U-CC), (E-CC;U-CT), (E-CC;U-TT), (E-CT;U-CC), (E-CT;U-CT), (E-TT;U-CC) and "other" combinations.

Tables summarizing analyses conducted using the UASMS2 and EXON2-FB markers individually are shown in Tables 1 and 2 respectively.

*Table 1: Results from Individual Analysis of the UASMS 2 Marker-Summary Statistics: Means by Genotype*

| | UASMS2 | | | |
|---|---|---|---|---|
| Variable | CC | CT | TT | P-value |
| *Model Input Variable* | | | | |
| Placement weight (lbs) | 695.890 | 687.761 | 654.854 | <0.01 |
| Ultrasound backfat (BF) at placement | 0.096 | 0.090 | 0.083 | <0.01 |
| Frame score at placement | 6.679 | 6.837 | 6.737 | 0.08 |
| Days on feed | 140.791 | 138.765 | 139.000 | 0.18 |
| Percent steer | 68.20 | 64.20 | 59.00 | 0.05 |
| Percent managed via backfat method | 28.90 | 24.60 | 18.80 | 0.02 |
| *Output Variables* | | | | |
| Percent choice (via MBS) | 45.60 | 41.90 | 41.70 | 0.30 |
| Marbling score (MBS) | 40.394 | 39.395 | 39.236 | 0.05 |
| Calculated yield grade | 2.747 | 2.769 | 2.725 | 0.75 |
| Dressing percentage | 63.70 | 63.39 | 63.06 | <0.01 |
| *Other Variables of Interest* | | | | |
| Plant backfat | 0.479 | 0.472 | 0.475 | 0.75 |
| Live weight at slaughter (lbs) | 1225.582 | 1209.880 | 1171.875 | <0.01 |
| AdjNR | -120.256 | -118.428 | -111.885 | 0.54 |

(continued)

| | UASMS2 | | | |
|---|---|---|---|---|
| Variable | CC | CT | TT | P-value |
| AdjRTR | 547.747 | 539.143 | 532.810 | 0.10 |
| Number of observations | 843 | 707 | 144 | |
| Percent of observations | 49.76 | 41.74 | 8.50 | |

*Table 2: Results from Individual Analysis of the EXON2-FB Marker-Summary Statistics: Means by Genotype*

| | EXON2-FB | | | |
|---|---|---|---|---|
| Variable | CC | CT | TT | P-value |
| *Model Input Variables* | | | | |
| Placement weight (lbs) | 688.953 | 690.864 | 686.433 | 0.79 |
| Ultrasound backfat (BF) at placement | 0.092 | 0.092 | 0.096 | 0.25 |
| Frame score at placement | 6.891 | 6.713 | 6.595 | 0.01 |
| Days on feed | 140.037 | 139.608 | 139.439 | 0.91 |
| Percent steer | 60.60 | 67.00 | 71.60 | <0.01 |
| Percent managed via backfat method | 24.10 | 26.10 | 29.90 | 0.17 |
| *Output Variables* | | | | |
| Percent choice (via MBS) | 43.60 | 42.60 | 47.00 | 0.41 |
| Marbling score (MBS) | 39.437 | 39.732 | 40.966 | 0.03 |
| Calculated yield grade | 2.691 | 2.739 | 2.900 | <0.01 |
| Dressing percentage | 63.56 | 63.56 | 63.37 | 0.32 |
| *Other Variables of Interest* | | | | |
| Plant backfat | 0.464 | 0.472 | 0.505 | <0.01 |
| Live weight at slaughter (lbs) | 1210.766 | 1218.300 | 1215.976 | 0.59 |
| AdjNR | -119.233 | -120.589 | -114.660 | 0.56 |
| AdjRTR | 549.951 | 542.572 | 534.758 | 0.09 |
| Number of observations | 535 | 816 | 328 | |
| Percent of Observations | 31.86 | 48.60 | 19.54 | |

*Table 3 reports summary statistics for the cattle used in this analysis broken down by genotype.*

| Variables | Genotypes | | | |
|---|---|---|---|---|
| | E[a]=CC; U[b]=CC | E=CC; U=CT | E=CC; U=TT | E=CT; U=CC |
| *Model Input Variables* | | | | |
| Placement weight (lbs) | 722.127 | 688.922 | 653.93 | 696.344 |
| Ultrasound backfat (BF) at placement | 0.102 | 0.092 | 0.080 | 0.095 |
| Frame score at placement | 6.788 | 6.991 | 6.797 | 6.695 |
| Days on feed | 138.493 | 141.546 | 138.664 | 142.385 |
| Percent steer | 67.20 | 58.40 | 57.80 | 66.10 |
| Percent managed via backfat method | 31.30 | 24.20 | 16.40 | 27.00 |
| *Output Variables* | | | | |
| Percent choice (via MBS) | 44.00 | 45.00 | 39.80 | 45.90 |
| Marbling score (MBS) | 39.761 | 39.665 | 38.672 | 40.365 |
| Calculated yield grade | 2.673 | 2.692 | 2.696 | 2.658 |
| Dressing percentage | 63.86 | 63.59 | 63.18 | 63.85 |
| *Other Variables of Interest* | | | | |
| Plant backfat | 0.465 | 0.462 | 0.467 | 0.464 |
| Live weight at slaughter (lbs) | 1245.075 | 1213.498 | 1168.547 | 1229.898 |
| AdjNR | -129.337 | -116.445 | -113.232 | -120.317 |
| AdjRTR | 566.785 | 549.224 | 535.019 | 550.876 |
| Number of observations | 134 | 269 | 128 | 392 |
| Percent of observations | 8.03 | 16.13 | 7.67 | 23.50 |

| Variables | Genotype | | | |
|---|---|---|---|---|
| | E=CT; U=CT | E=TT; U=CC | All other combos[c] | P-value[d] |
| *Model Input Variable (at placement)s* | | | | |
| Placement weight (lbs) | 685.578 | 684.377 | 708.034 | <0.01 |
| Ultrasound backfat (BF) at placement | 0.089 | 0.096 | 0.107 | <0.01 |
| Frame score at placement | 6.745 | 6.622 | 6.282 | 0.02 |
| Days on feed | 137.096 | 139.815 | 134.793 | 0.02 |
| Percent steer | 67.60% | 70.80% | 79.30% | 0.01 |
| Percent managed via backfat method | 24.80% | 29.90% | 37.90% | 0.04 |
| *Output Variables* | | | | |
| Percent choice (via MBS) | 39.20% | 46.40% | 58.60% | 0.2 |
| Marbling score (MBS) | 38.941 | 40.821 | 44.31 | <0.01 |
| Calculated yield grade | 2.810 | 2.894 | 3.076 | <0.01 |
| Dressing percentage | 63.30% | 63.41% | 62.81% | <0.01 |
| *Other Variables of Interest* | | | | |
| Plant backfat | 0.477 | 0.503 | 0.546 | <0.01 |
| Live weight at slaughter (lbs) | 1206.581 | 1213.354 | 1238.448 | <0.01 |
| AdjNR | -121.139 | -116.102 | -93.02 | 0.39 |
| AdjRTR | 534.904 | 535.866 | 520.376 | <0.01 |
| Number of observations | 408 | 308 | 29 | |
| Percent of observations | 24.46% | 18.47% | 1.74% | |

[a]E represents the EXON2-FB marker; [b]U represents the UASMS2 marker; [c]All other EXON2-FB /UASMS2 combinations (of the 29 animals in this category, 18 are E=TT;U=

CT, 1 is E=TT;U=TT, and 10 are E=CT;U=TT); [d]P-value associated with an ANOVA test that the means are equivalent across genetic types.

Most of the cattle in the 1,668 population of the study group had the (E-CT;U-CT) or (E-CT;U-CC) genotypes (48% of the cattle in the sample). Only 29 animals (1.7% of the sample) were of "other" genotypes. For almost every variable shown in Table 1, the hypothesis that means are equivalent for all seven genotypes can be rejected at the P=0.05 level or lower.

Although only comprising 1.7% of the sample, "other" cattle tended to be the fattest. They also had higher marbling scores, higher yield grades, and lower dressing percentages compared to all other genotypes. (E-TT;U-CC) cattle had the second highest average marbling score and yield grade. (E-CC;U-TT) had the lowest mean marbling scores and (E-CT;U-CC) cattle had the lowest mean yield grade. (E-CC;U-CC) cattle had the highest average live weight at slaughter, which may be due to this genotype also having the highest average placement weight. (E-CC;U-CC) cattle had the highest mean adjusted rate of return (AdjRTR).

### Example 2: Price Data Used in Marketing Simulation Experiments

To avoid potential time-varying effects that may have occurred in the market, it was assumed that all 1,668 test animals were sold and marketed on the same date under the same market prices. Weekly live and dressed weight prices, reported as the five-market weighted average by the USDA-AMS, were averaged for the year 2004 for use in this analysis. Weekly grid premiums and discounts were obtained from the USDA-AMS National Carlot Meat Report for the year 2004. The average premiums and discounts reported from various packers to the USDA-AMS over this time period were also used. The prices used in the analysis are shown in Table 4.

Table 4: Price Data Used in Simulation Analysis (Based on USDA/AMS data for 2004)

| Marketing Method | | Price ($/cwt) | |
|---|---|---|---|
| Grid | Base Price[a] | $118.00 | |
| | Quality Grade Adjustment | | |
| | Prime | $8.29 | |
| | Choice | $0.00 | |
| | Select | -$8.72 | |
| | Standard | -$18.25 | |
| | Yield Grade Adjustment | | |
| | 1.0 - 2.0 | $2.93 | |
| | 2.0 - 2.5 | $1.67 | |
| | 2.5 - 3.0 | $1.24 | |
| | 3.0 - 4.0 | -$0.08 | |
| | 3.5 - 4.0 | -$0.08 | |
| | 4.0 - 5.0 | -$13.70 | |
| | >5.0 | -$18.04 | |
| | Carcass Weight Adjustment (lbs) | | |
| | <500 | -$21.69 | |
| | 500 - 550 | -$14.98 | |
| | 950 - 1000 | -$7.44 | |
| | >1000 | -$18.04 | |
| Live weight[b] | | $72.66 | |

(continued)

| Marketing Method | | | Price ($/cwt) | |
|---|---|---|---|---|
| Dressed weight[b] | | | $114.43 | |

[a]Grid base price calculated based on the formula: Grid Base Price = (dressed weight cash price) + [(Choice-to-Select price spread) x (plant average percent Choice)]; [b]based on the five market weighted average as reported by USDA/AMS in 2004. The base price used for the grid was calculated as:

$$\text{grid base price} = (\text{dressed weight cash price}) + [(\text{Choice-to-Select price spread}) \times (\text{plant average percent Choice})]$$

where the feedlot plant average percent Choice was set equal to the percentage Choice in this particular data set.

## Example 3: Simple Marketing Simulations

(a) The revenue that would be obtained if all 1,668 test cattle were marketed on a live weight basis was calculated. Live weight revenue for each animal was calculated as live weight of the animal at slaughter multiplied by the live weight price shown in Table 2. To determine which genotype would generate the highest revenue on a live weight basis, the mean revenue per head for cach genotype, on a live weight basis, was calculated.

(b) The revenue that would be obtained if all 1,668 test head were marketed on a dressed weight basis was determined. Dressed weight revenue for each animal was calculated as the dressed weight of the animal at slaughter multiplied by the live weight price shown in Table 2. Dressed weight revenues were broken down by genotype to determine which genotype generated the highest revenue on a dressed weight basis.

(c) The revenue obtained from marketing all 1,668 test head on a grid was determined by utilizing each animal's quality characteristics and the grid price data shown in Table 2. As with the two marketing methods (a) and (b), the mean revenue per head for each genotype was calculated for the grid marketing method to determine which genotype performed best based on a grid. Tables 4 and 5 show the results from the marketing simulations.

*Table 4: Results from Basic Simulation (no predictive model is used)*

| Revenue ($/head) from ... | N | Mean Revenue ($/head) | St. Dev | Min Revenue ($/head) | Max Revenue ($/head) |
|---|---|---|---|---|---|
| **Market all Live Weight** | | | | | |
| Total Cattle | 1,668 | 882.93 | 95.45 | 662.66 | 1158.93 |
| Market all Exon-CC; UASMS2-CC | 134 | 904.67 | 93.14 | 663.39 | 1098.62 |
| Market all Exon-CC; UASMS2-CT | 269 | 881.73 | 91.34 | 668.47 | 1078.27 |
| Market all Exon-CC; UASMS2-TT | 128 | 849.07 | 100.10 | 662.66 | 1065.92 |
| Market all Exon-CT; UASMS2-CC | 392 | 893.64 | 93.99 | 675.01 | 1101.53 |
| Market all Exon-CT; UASMS2-CT | 408 | 876.70 | 93.64 | 665.57 | 1158.93 |
| Market all Exon-TT; UASMS2-CC | 308 | 881.62 | 98.42 | 683.73 | 1128.41 |
| "other" | 29 | 899.86 | 94.35 | 706.26 | 1044.12 |
| **Market all Dressed Weight** | | | | | |
| Total Cattle | 1,668 | 883.16 | 98.59 | 604.19 | 1187.78 |

(continued)

| Revenue ($/head) from ... | N | Mean Revenue ($/head) | St. Dev | Min Revenue ($/head) | Max Revenue ($/head) |
|---|---|---|---|---|---|
| Market all Exon-CC; UASMS2-CC | 134 | 910.19 | 102.09 | 625.93 | 1122.56 |
| Market all Exon-CC; UASMS2-CT | 269 | 882.51 | 91.01 | 646.53 | 1090.52 |
| Market all Exon-CC; UASMS2-TT | 128 | 844.61 | 101.24 | 604.19 | 1101.96 |
| Market all Exon-CT; UASMS2-CC | 392 | 898.49 | 97.18 | 629.37 | 1187.78 |
| Market all Exon-CT; UASMS2-CT | 408 | 874.11 | 98.23 | 644.24 | 1172.91 |
| Market all Exon-TT; UASMS2-CC | 308 | 879.87 | 98.86 | 661.41 | 1147.73 |
| "other" | 29 | 889.67 | 95.71 | 702.60 | 1033.30 |
| **Market all on Grid** | | | | | |
| Total Cattle | 1,668 | 874.37 | 104.00 | 524.96 | 1134.47 |
| Market all Exon-CC; UASMS2-CC | 134 | 896.48 | 96.56 | 524.96 | 1106.51 |
| Market all Exon-CC; UASMS2-CT | 269 | 879.38 | 99.92 | 600.01 | 1100.59 |
| Market all Exon-CC; UASMS2-TT | 128 | 834.49 | 108.35 | 550.49 | 1091.39 |
| Market all Exon-CT; UASMS2-CC | 392 | 892.43 | 102.14 | 617.16 | 1127.29 |
| Market all Exon-CT; UASMS2-CT | 408 | 862.33 | 103.25 | 577.30 | 1134.47 |
| Market all Exon-TT; UASMS2-CC | 308 | 869.27 | 105.68 | 595.76 | 1116.70 |
| "other" | 29 | 881.19 | 104.20 | 640.40 | 1060.56 |

*Table 5. Summary of Basic Simulation Results in Table 4[a]*

| Genotype | Best Marketing Method | Mean Revenue ($/head) when Marketed by Best Method | Ranking |
|---|---|---|---|
| Exon-CC; UASMS2-CC | dressed weight | 910.19 | 1 |
| Exon-CC; UASMS2-CT | dressed weight | 882.51 | 4 |
| Exon-CC; UASMS2-TT | live weight | 849.07 | 7 |
| Exon-CT; UASMS2-CC | dressed weight | 898.49 | 3 |
| Exon-CT; UASMS2-CT | live weight | 876.70 | 6 |
| Exon-TT; UASMS2-CC | live weight | 881.62 | 5 |
| "other" | live weight | 899.86 | 2 |
| [a]Results in this table assume all animals of the same genotype are sold by the same marketing method | | | |

Results show that if all 1,668 head are marketed on a dressed weight basis, average revenues would be $883.16/head. This figure would fall to $882.93 if all cattle were marketed on a live weight basis and $874.37 if all cattle were marketed on a grid.

Regardless of the marketing method, (E-CC;U-CC) cattle would provide the highest revenue of all seven genotypes. Looking across marketing methods, (E-CC;U-CC) cattle would provide the highest revenue when marketed on a dressed weight basis at $910.19/head. In contrast, regardless of marketing method, (E-CC;U-TT) cattle would provide the lowest revenue of all seven genotypes. Even if (E-CC;U-TT) were marketed by live weight (the method providing the highest revenue for this genotype), revenues would be only $849.07. Thus, there would be a substantial difference of $61.12/head between the best and worst genotypes.

The data also indicate that genotypes (E-CC;U-CC), (E-CC;U-CT) and (E-CT;U-CC) would best be marketed on a dressed weight basis, whereas the other four genotypes would be best marketed on a live weight basis.

The difference between marketing a genotype by its best method versus the second best method would not be inconsequential. For example, while (E-CC;U-CC) cattle would generate $910.19/head revenue when marketed on a dressed weight basis, they would only generate $904.67/head when marketed on a live weight basis - a difference of $5.52/head. For this particular set of cattle and the particular prices used in the simulation, it would never be optimal to market all animals of a given genotype on a grid determnation.

If all animals of a given genotype, therefore, were marketed by the *same* marketing method, this analytical method indicates which marketing method would provide the highest revenue for each genotype. Once it is determined which marketing method provides the highest revenue *for each genotype,* it can be determined which of the seven genotypes provides the highest overall revenue when each genotype is being "optimally" marketed.

### Example 4: Advanced Marketing Simulations Based on Predictive Models

To optimally determine how an animal should be marketed, data gathered at placement were used to predict these slaughter characteristics. An animal's yield grade, quality grade, and dressing percentage, therefore, need to be forecast. In this analysis, genotype, backfat ultrasound measurements and other animal characteristics garnered *prior* to placement were used to predict three final carcass characteristics: quality grade, marbling, and dressing percentage at slaughter time. Using this approach, animals could be appropriately sorted upon delivery to a feedlot for more tailored feeding schedules.

These dependent variables are continuous in nature, and therefore ordinary least squares regression was used to estimate the prediction models. The explanatory variables included in the models were an animal's placement weight, ultrasound backfat measure at placement, frame score at placement, days on feed, a dummy variable distinguishing steers from heifers, and six dummy variables identifying the effect of each of the genotypes relative to the seventh genotype "other." Table 6 reports the estimated prediction models.

*Table 6. Yield Grade, Marbling Score, and Dressing Percentage Models (N = 1,668 in each regression)*

| Independent Variables | Fully Specified Models | | |
|---|---|---|---|
| | Yield Grade | Marb. Score | Dress. Percent |
| Intercept | 3.255**[a] | 37.305** | 59.262** |
| | (0.270)[b] | (3.364) | (0.805) |
| Placement weight (lbs) | 0.0002 | 0.002 | 0.003** |
| | (0.0002) | (0.003) | (0.001) |
| Ultrasound backfat at placement | 4.611** | 33.288** | 6.376** |
| | (0.625) | (7.789) | (1.863) |
| Frame score at placement | -0.040* | 0.058 | -0.135* |
| | (0.019) | (0.241) | (0.058) |
| Days on feed | -0.003** | 0.023* | 0.016** |
| | (0.001) | (0.011) | (0.003) |
| Steer (1 = steer; 0 =heifer) | -0.158** | -2.077** | -0.661* |
| | (0.061) | (0.760) | (0.182) |
| Method of managing cattle[c] | 0.143** | 1.240 | 0.122 |
| | (0.056) | (0.695) | (0.166) |
| E=CC;U=CC[d] | -0.361** | -4.705** | 0.974' |
| | (0.137) | (1.704) | (0.408) |
| E=CC;U=CT[d] | -0.274* | -4.575** | 0.798* |
| | (0.131) | (1.628) | (0.389) |
| E-CC;U-TT[d] | -0.218 | -4.960" | 0.587 |

(continued)

| Independent Variables | Fully Specified Models | | |
|---|---|---|---|
| | Yield Grade | Marb. Score | Dress. Percent |
| | (0.138) | (1.715) | (0.410) |
| E=CT;U=CC[d] | -0.322* | -3.852* | 1.017** |
| | (0.129) | (1.601) | (0.383) |
| E=CT;U=CT[d] | -0.152 | -4.889** | 0.634 |
| | (0.128) | (1.599) | (0.382) |
| E=TT; U=CC[d] | -0.100 | -3.308* | 0.651 |
| | (0.130) | (1.615) | (0.386) |
| $R^2$ | 0.19 | 0.08 | 0.08 |
| Independent Variables | Models w/ Genotype Only | | |
| | Yield Grade | Marb. Score | Dress. Percent |
| Intercept | 3.076** | 44.310** | 62.808** |
| | (0.136) | (1.594) | (0.381) |
| Placement weight | - | - | - |
| Ultrasound backfat at placement | - | - | - |
| Frame score at placement | - | - | - |
| Days on feed | - | - | - |
| Steer (1 = steer; 0 =heifer) | - | - | - |
| Method of managing cattle[c] | - | - | - |
| E=CC;U=CC[d] | -0.403** | -4.549** | 1.049* |
| | (0.150) | (1.758) | (0.421) |
| E=CC;U=CT[d] | -0.383** | -4.645** | 0.783* |
| | (0.143) | (1.677) | (0.401) |
| E=CC;U=TT[d] | -0.380* | -5.638** | 0.372 |
| | (0.150) | (1.765) | (0.422) |
| E=CT;U=CC[d] | -0.418** | -3.946* | 1.045** |
| | (0.141) | (1.652) | (0.395) |
| E=CT;U=CT[d] | -0.265 | -5.369** | 0.490 |
| | (0.141) | (1.649) | (0.395) |
| E=TT; U=CC[d] | -0.181 | -3.489* | 0.597 |
| | (0.142) | (1.667) | (0.399) |
| $R^2$ | 0.02 | 0.01 | 0.02 |
| Independent Variables | Models w/ Ultrasound Only | | |
| | Yield Marb. Grade Score | | Dress. Percent |
| Intercept | 2.171** | 36.022** | 41.566** |
| | (0.037) | (0.461) | (4.440) |
| Placement weight | - | - | - |
| Ultrasound backfat at placement | 6.305** | 41.566** | 8.347** |
| | (0.358) | (4.440) | (1.073) |
| Frame score at placement | - | - | - |

(continued)

| Independent Variables | Fully Specified Models | | |
|---|---|---|---|
| | **Yield Grade** | **Marb. Score** | **Dress. Percent** |
| Days on feed | - | - | - |
| Steer (1 = steer; 0 =heifer) | - | - | - |
| Method of managing cattle[c] | - | - | - |
| E=CC;U=CC[d] | - | - | - |
| E=CC;U=CT[d] | - | - | - |
| E=CC;U=TT[d] | - | - | - |
| E=CT;U=CC[d] | - | - | - |
| E=CT;U=CT[d] | - | - | - |
| E=TT; U=CC[d] | - | - | - |
| $R^2$ | 0.16 | 0.05 | 0.04 |

[a]One (*) and two (**) asterisks represent 0.05 and 0.01 levels of statistical significance, respectively; [b]Numbers in parentheses are standard errors of the coefficients; [c]Takes the value of 1 if feedlot used BF (backfat) method; 0 otherwise; [d]Effects of all other genotypes estimated relative to this "other" category.

The first three columns report results for the fully specified models for yield grade, marbling score, and dressing percentage, respectively and show that an increased level of backfat at placement is associated with significantly higher yield grades, marbling scores, and dressing percentages. For a one inch increase in backfat at placement, yield grade increases by 4.6, marbling score increases by 33.3, and dressing percent increases by 6.4%.

More days on feed are associated with higher marbling scores, and higher dressing percentages. For each extra day on feed, one can expect the marbling score to increase by 0.023.

The last seven variables in Table 6 are dummy variables for identifying the effect of genotype and indicate that on average (E-CC;U-CC) cattle have yield grades 0.36 lower, marbling scores 4.71 lower, and dressing percentages about 1% higher than "other" cattle. Animals of the "other" genotypes tend to have higher yield grades, higher marbling scores, and lower dressing percentages than the other genotypes.

Some genotypes such as (E-CC;U-TT) and (E-CT;U-CT) and (E-TT;U-CC) are not significantly different than "other" cattle with respect to yield grade and dressing percentage, once differences in other cattle characteristics are controlled for. The rest of the results in Table 6 are associated with the models that either include only genotypic information or only ultrasound backfat measures.

## Example 5

Actual animal characteristics may differ from predicted or expected animal characteristics. In such cases, an animal may not be marketed by a method that provides the highest revenue. Therefore, to provide information about the *potential* of *perfect* prediction equations, steps (a)-(d) as presented above would be followed but *actual,* rather than *predicted,* slaughter characteristics may be used. That is, the actual revenue for each animal may be calculated when each animal has been sold under the method providing the highest revenue given an animals carcass characteristics. Finally, to assess the value that genetic information provides to the prediction equations relative to other information, the simulation was repeated with predictive models that use: a) only the genotype dummy variables, and b) only the ultrasound backfat measure. Table 7 shows the results of the simulations that use the predictive models to market each animal by the method expected to provide the highest revenue.

*Table 7: Results from Simulation Using Predictive Models*

| Revenue ($/head) from... | N | Mean Revenue ($/head) | St. Dev | Min Revenue ($/head) | Max Revenue ($/head) |
|---|---|---|---|---|---|
| Using models with genotype only as shown in Table 4 to choose marketing Method | 1,668 | 884.31 | 97.27 | 625.93 | 1,187.78 |
| Using models with ultrasound information only as shown in Table 4 to choose marketing method | 1,668 | 884.50 | 97.75 | 636.23 | 1,187.78 |
| Using the fully specified models shown in Table 4 to choose marketing method | 1,668 | 885.92 | 98.53 | 640.40 | 1,187.78 |
| Maximum possible revenue; using a model with perfect predictions[a] | 1,668 | 905.02 | 99.32 | 662.66 | 1,187.78 |
| [a]If perfect information is known about cattle characteristics, it would be optimal to market 32.9% of cattle on live weight basis, 31.4% of cattle on dressed weight basis, and 35.7% of cattle on a grid basis. | | | | | |

The fully specified models shown in Table 7 show that average revenues of $885.92/head are obtainable, which generates $2.76, $2.99, and $11.55/head more revenue than naïvely marketing all 1,668 animals by dressed weight, live weight, or grid, respectively. Models that use only genotypic information or only backfat measures perform worse than the fully specified models. However, they still perform better than naïve marketing strategies where all animals are marketed under a single marketing method.

The last row in Table 7 shows that the maximum possible revenue is $905.02/head if each animal were marketed via the method actually providing the highest revenue. Furthermore, this figure is over $5/head *less* than average revenues for (E-CC;U-CC) cattle when those cattle are marketed dressed weight (see Table 6), illustrating that of the 1,668 cattle in the data set, many arc poor performing and there are substantial returns to be realized by only feeding cattle of certain genotypes.

As shown in Table 7, if each animal were marketed in the method actually providing the highest revenue, average revenue would be $905.02/head. Table 8 breaks down this maximum obtainable revenue by genotype.

*Table 8: Summary of Simulation Where Each Animal is Marketed by the Method Providing the Highest Revenue*

| Genotype | Percent Optimally Marketed by Live Weight | Percent Optimally Marketed by Dressed Weight | Percent Optimally Marketed by Grid | Mean Revenue ($/head) if Optimally Marketed | Ranking |
|---|---|---|---|---|---|
| Exon-CC; UASMS2-CC | 26.87 | 37.31 | 35.82 | 931.128 | 1 |
| Exon-CC; UASMS2-CT | 27.88 | 32.71 | 39.41 | 905.137 | 4 |
| Exon-CC; UASMS2-TT | 42.19 | 25.78 | 32.03 | 867.290 | 7 |
| Exon-CT; UASMS2-CC | 26.79 | 33.67 | 39.54 | 919.981 | 2 |

(continued)

| Genotype | Percent Optimally Marketed by Live Weight | Percent Optimally Marketed by Dressed Weight | Percent Optimally Marketed by Grid | Mean Revenue ($/head) if Optimally Marketed | Ranking |
|---|---|---|---|---|---|
| Exon-CT; UASMS2-CT | 36.76 | 31.13 | 32.11 | 894.629 | 6 |
| Exon-TT; UASMS2-CC | 37.34 | 29.22 | 33.44 | 902.649 | 5 |
| "other" | 44.83 | 17.24 | 37.93 | 918.906 | 3 |

As shown in Table 8, if each animal of the (E-CC;U-CC) genotype were marketed by the method actually generating the highest revenue, 26.87% would be marketed live weight, 37.31% would be marketed dressed weight, and 35.82% would be marketed on a grid, with the end result being an average revenue of $931.13/head. The ranking of genotypes by revenues are almost equivalent in Tables 5 and 8. Therefore, regardless of whether all animals of a genotype must be marketed together by the same method or whether each animal is individually marketed regardless of genotype, (E-CC;U-CC), (E-CT;U-CC) and "other" cattle provide the highest revenues, whereas (E-CC; U-TT) cattle provide the lowest revenues.

### Example 6: Marker Combination Frequencies

Table 9 illustrates the genotype and allele frequencies for the ob gene markers UASMS1, UASMS2 and EXON2-FB among a population of test cattle

*Table 9: Genotype and allele frequencies*

| Marker | Genotype | Number | Frequency Genotype | Allele |
|---|---|---|---|---|
| UASMS1 | CC | 380 | 0.19 | 0.43 |
| | CT | 935 | 0.48 | |
| | TT | 639 | 0.33 | 0.57 |
| UASMS2 | CC | 977 | 0.50 | 0.71 |
| | CT | 809 | 0.41 | |
| | TT | 168 | 0.08 | 0.29 |
| EXON2-FB | CC | 638 | 0.33 | 0.57 |
| | CT | 940 | 0.48 | |
| | TT | 376 | 0.19 | 0.43 |

Table 10 illustrates the frequencies of the haplotypes of each pair of single nucleotide polymorphisms of the promoter region EXON2fb of the ob gene in the population of test cattle.

*Table 10: Marker Combination Frequencies*

| Combination | Haplotype C-C | C-T | T-C | T-T |
|---|---|---|---|---|
| UASMS1 & UASMS2 | .435 | .002 | .267 | .296 |
| UASMS1 & EXON2-FB | .025 | .414 | .535 | .026 |
| UASMS2 & EXON2-FB | .281 | .421 | .279 | .018 |

Table 11 illustrates the frequencies of the individual genotypes for the two polymorphic sites UASMS2 and EXON2-FB of the *ob* gene.

*Table 11: Genotype frequencies for UASMS2 & EXON2fb*

| | | EXON2-FB | | | | |
|---|---|---|---|---|---|---|
| | CC | | CT | | TT | |
| | N | Freq | N | Freq | N | Freq |
| CC | 136 | 7.71 | 415 | 23.53 | 321 | 18.2 |
| CT | 288 | 16.33 | 426 | 24.15 | 25 | 1.42 |
| TT | 136 | 7.71 | 15 | 0.85 | 2 | 0.11 |

## Example 7: Significant 2-Marker Associations

1,633 cattle records were obtained. Significant marker associations were found for the UASMS2-EXON2-FB (EXON2-FB) polymorphic site pair with the characters hot carcass weight (HCW), rib-eye area (REA), DP, body fat (BFAT), yield grade (YG), MBS ad MBS?DOF.. The results, illustrated in Figs 1-6, indicate that the greatest effects impacting the economically important characters of Hot carcass Weight, Ribeye Area, Backfat, Yield Grade, Dressing Percentage and Marbling respectively are associated with the UASMS2 & EXON2-FB marker genotype CC-CC.

## Example 8: Backfat Prediction Model

The following model was used as a basis for estimation (Brethour. J. Anim. Sci. 78: 2055-61 (2000))

$$Y = Ae^{kt}$$

Where, Y is projected backfat at time $t$, $A$ = backfat thickness at placement, $k$ = a parameter to be estimated representing the rate of increase in backfat, and $t$ = days on feed. Instead of estimating one parameter, $k$, for the entire dataset, $k$ is parameterized as follows:

$$k = b_o + b_1 type_1 + b_2 type_2 + b_3 type_7 + b_4 type_4 + b_5 type_5 + b_6 type_6 + g_1 steer + g_2 bfmethod + g_3 plcwt + g_4 frame1$$

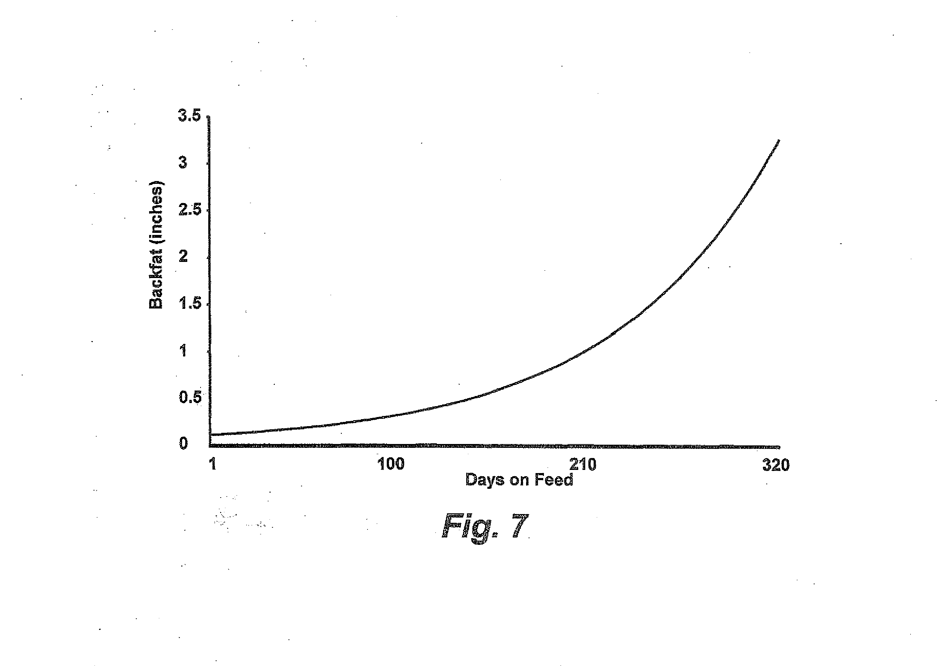

such that the rate of increase in backfat depends on genotype, gender *(steer),* whether the animal was fed (attempted at least) to a constant backfat *(bfmethod),* placement weight *(plcwt),* and frame score at placement *(frame1).* $type_1$ = EXON2-FB CC & UASMS2 CC, $type_2$ = EXON2-FB CC & UASMS2 CT, $type_3$ = EXON2-FB CC & UASMS2 TT, $type_4$ = EXON2-FB CT & UASMS2 CC, $type_5$ = EXON2-FB CT & UASMS2 CT, $type_6$ = EXON2-FB TT & UASMS2 CC, $type_7$ = all other EXON/UASMS2 combinations. Estimation Results are shown in Table 12 and Fig. 7.

*Table 12: Nonlinear OLS Summary of Residual Errors*

| Equation | DF Model | DF Error | SSE 19.6981 | MSE 0.00586 | Root MSE | R-Square | Adj R-Sq |
|---|---|---|---|---|---|---|---|
| bfstar | 11 | 3361 | | | 0.0766 | 0.6867 | 0.6858 |
| | Nonlinear OLS Parameter Estimates | | | | | | |
| Parameter | Estimate | | Approx Std Err | | t Value | Approx Pr > |t| | |
| b0 | 0.012249 | | 0.000502 | | 24.41 | <.0001 | |
| b1 | -0.00145 | | 0.000268 | | -5.41 | <.0001 | |
| b2 | -0.00153 | | 0.000229 | | -6.66 | <.0001 | |
| b3 | -0.00076 | | 0.000409 | | -1.87 | 0.0615 | |
| b4 | -0.0016 | | 0.000215 | | -7.47 | <.0001 | |
| b5 | -0.00058 | | 0.000215 | | -2.70 | 0.0070 | |
| b6 | -0.00127 | | 0.000221 | | -5.75 | <.0001 | |

(continued)

| Equation bfstar | DF Model 11 | DF Error 3361 | SSE 19.6981 | MSE 0.00586 | Root MSE 0.0766 | R-Square 0.6867 | Adj R-Sq 0.6858 |
|---|---|---|---|---|---|---|---|
| | Nonlinear OLS Parameter Estimates | | | | | | |
| g1 steer | 0.002282 | | 0.000179 | | 12.75 | | <.0001 |
| g2 bfmethod | -0.0023 | | 0.000114 | | -20.17 | | <.0001 |
| g3 plcwt | -0.00861 | | 0.000574 | | -14.98 | | <.0001 |
| g4 frame | 0.000731 | | 0.000059 | | 12.35 | | <.0001 |
| (Note: these estimates do not include the final backfat measures at slaughter; the $R^2$ from a model that includes all backfat measures is only .5088) | | | | | | | |

## Example 9: Marbling Score Prediction Model

The following model was used as a basis for estimation (Brethour. J. Anim. Sci. 78: 2055-61 (2000))

$$Y = Ae^{kt}$$

Where, $Y$ is projected marbling score at time $t$, $A$ = marbling score at placement, $k$ = a parameter to be estimated representing the rate of increase in marbling, and $t$ = days on feed.

Again, rather than estimating one parameter, $k$, for the entire dataset, $k$ is instead parameterized as follows:

$$k = b_o + b_1 type_1 + b_2 type_2 + b_3 type_7 + b_4 type_4 + b_5 type_5 + b_6 type_6 + g_1 steer + g_2 bfmethod + g_3 plcbfat + g_4 frame1 + g_5 plcwt$$

such that the rate of increase in marbling depends on genotype, gender (*steer*), whether the animal was fed (attempted at least) to a constant backfat (*bfmethod*), ultrasound backfat at placement (*plcbfat*), placement weight (*plcwt*), and frame score at placement (*frame1*).

In this particular data set, there were no ultrasound measures of marbling at placement (e.g., $A$ in the equation above). Thus, the marbling models estimated by Brethour (J. An. Sci, 2000) were used, appropriately adjusting for differences in the way marbling was measured, to "backcast" the marbling score at placement. In particular, Brethour estimated the following model (using his notation): $Y = I + kt^m$, where $I$, $k$, and $m$ are parameters and $t$ is days on feed. Using two datasets, Brethour estimated $I$ at 3.10 and 3.39, $k$ at 0.00214 and 1.23642 x $10^{-9}$, and $m$ at 1.55 and 3.42. Iinitial marbling at placement (*mbs1*) is equal to:

$$mbs1 = \exp(\log(\exp(\log(-(-mbsF + I)/k)/m)-t)*m)*k + I$$

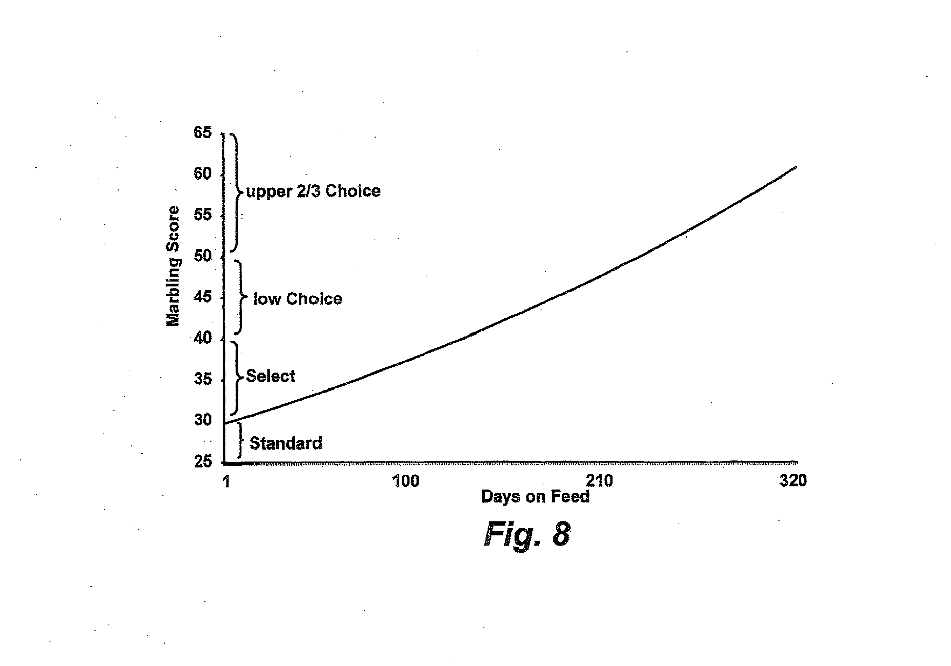

where *mbsF* is the final marbling score at slaughter. Using these estimates *mbs1* is calculated for each animal in the data set (the average prediction from the two estimates above is used) and this new variable is used to estimate the model above (note: $A = mbs1$) and as shown in Fig. 8.

*Table 13: Estimation Results-Nonlinear OLS Summary of Residual Errors*

| Equation MBS | DF Model 12 | DF Error 1626 | SSE 3599.7 | MSE 2.2138 | R-Square 0.9698 | Adj R-Sq 0.9696 |
|---|---|---|---|---|---|---|
| | Nonlinear OLS Parameter Estimates | | | | | |
| Parameter | | Estimate | Approx Std Err | | t Value | Approx Pr > \|t\| |
| b0 | | 0.001931 | 0.000065 | | 29.90 | <.0001 |

(continued)

| Equation MBS | DF Model 12 | DF Error 1626 | SSE 3599.7 | MSE 2.2138 | R-Square 0.9698 | Adj R-Sq 0.9696 |
|---|---|---|---|---|---|---|
| | | Nonlinear OLS Parameter Estimates | | | | |
| Parameter | | Estimate | Approx Std Err | | t Value | Approx Pr > \|t\| |
| b1 | | -0.00001 | 0.000034 | | -0.36 | 0.7214 |
| b2 | | -3.02E-6 | 0.000029 | | -0.10 | 0.9171 |
| b3 | | 0.000139 | 0.000052 | | 2.70 | 0.0070 |
| b4 | | 0.000026 | 0.000027 | | 0.96 | 0.3355 |
| b5 | | 4.12E-8 | 0.000028 | | 0.00 | 0.9988 |
| b6 | | 0.000046 | 0.000028 | | 1.63 | 0.1031 |
| g1 steer | | -0.00004 | 0.000026 | | -1.60 | 0.1088 |
| g2 bfmethod | | 0.000029 | 0.000021 | | 1.39 | 0.1633 |
| g3 plcbfat | | 0.001037 | 0.000240 | | 4.32 | <.0001 |
| g4 frame1 | | 0.000011 | 8.217E-6 | | 1.28 | 0.2009 |
| g5 plcwt | | 1.865E-7 | 9.357E-8 | | 1.99 | 0.0465 |

## Example 10: Weight Prediction Model

To estimate live weight, a growth model was estimated. The final specification follows Kaps et al. J. Anim. Sci. 77: 569-74 (1999)) and is considered a "Brody" growth curve. The following model is used as a basis for estimation:

$$W = A - (A - W_0)e^{-kt}$$

Where $W$ is live weight at time $t$, $A$ is a parameter that represents asymptotic mature weight (e.g., maximum attainable weight), $W_0$ is a parameter that represents placement weight, and $k$ is a parameter representing the ration of maximum growth rate to mature size referred to as a maturing rate index.

As before, a more general specification is estimated. In particular, $A$, $W_0$ and $k$ are parameterized as follows:

$$A = a_o + a_1 type_1 + a_2 type_2 + a_4 type_4 + a_5 type_5 + a_6 type_6 + a_7 type_7 + a_8 steer + a_9 bfmethod + a_{10} frame1 + a_{11} plcbfat$$

$$W_0 = b_o + b_1 type_1 + b_2 type_2 + b_4 type_4 + b_5 type_5 + b_6 type_6 + b_7 type_7$$

$$k = c_o + c_1 type_1 + c_2 type_2 + c_4 type_4 + c_5 type_5 + c_6 type_6 + c_7 type_7 + c_8 steer + c_9 bfmethod + c_{10} frame1 + c_{11} plcbfat$$

where all variable definitions are the same as in Examples 5 and 6 above. In the simulation, the parameters in the $W_0$ equation are replaced with inputted placement weights for each genotype.

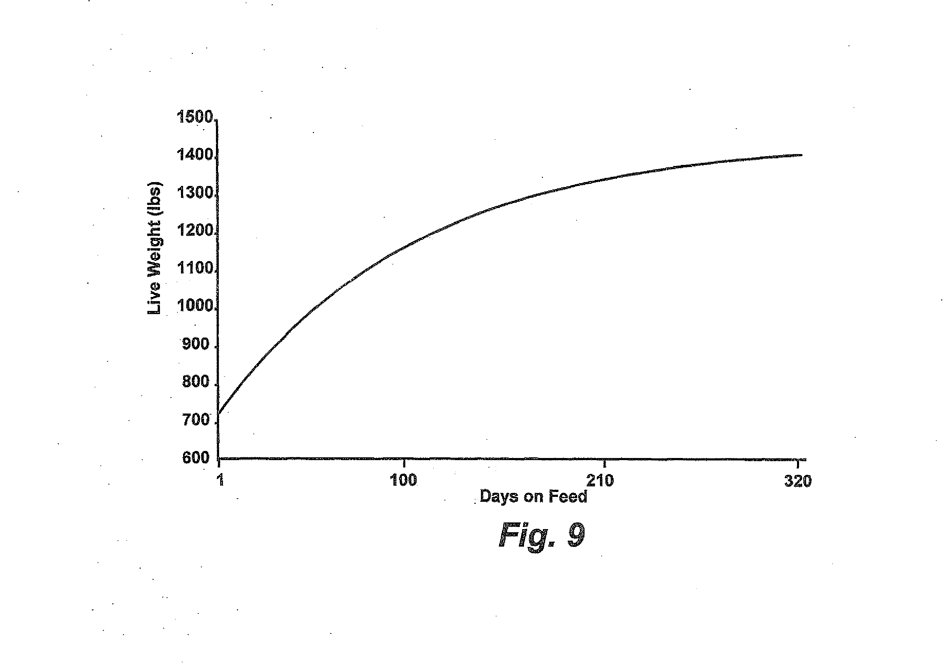

Estimation results are shown in Table 14 and Fig. 9.

*Table 14: Estimation Results- Nonlinear OLS Summary of Residual Errors*

| Equation | | DF Error | SSE | | Root MSE | R-Square | Adj R-Sq |
|---|---|---|---|---|---|---|---|
| wtstar | DF Model 29 | 5427 | 48146803 | MSE 8871.7 | 94.1898 | 0.8694 | 0.8687 |
| | Nonlinear OLS Parameter Estimates | | | | | | |
| Parameter | Estimate | | Approx Std Err | | t Value | | Approx Pr> \|t\| |
| a0 | -763.017 | | 67.8271 | | -11.25 | | <.0001 |
| a1 | 38.24259 | | 17.4630 | | 2.19 | | 0.0286 |
| a2 | 66.76775 | | 15.5808 | | 4.29 | | <.0001 |
| a4 | 26.99379 | | 14.4799 | | 1.86 | | 0.0623 |
| a5 | 24.4161 | | 14.1024 | | 1.73 | | 0.0834 |
| a6 | 12.50331 | | 14.4346 | | 0.87 | | 0.3864 |
| a7 | -18.0334 | | 28.9936 | | -0.62 | | 0.5340 |
| a8 steer | 762.3329 | | 27.2922 | | 27.93 | | <.0001 |
| a9 method1b | 2.87766 | | 11.3621 | | 0.25 | | 0.8001 |
| a 10 frame | 217.3536 | | 8.8786 | | 24.48 | | <.0001 |
| a11 bfat1 | 3136.082 | | 158.2 | | 19.82 | | <.0001 |
| b0 | 648.9606 | | 8.5052 | | 76.30 | | <.0001 |
| b1 | 64.17898 | | 11.8464 | | 5.42 | | <.0001 |
| b2 | 35.30738 | | 10.2832 | | 3.43 | | 0.0006 |
| b3 | 57.52833 | | 19.5748 | | 2.94 | | 0.0033 |
| b4 | 41.66914 | | 9.7491 | | 4.27 | | <.0001 |
| b5 | 29.69344 | | 9.7133 | | 3.06 | | 0.0022 |
| b6 | 27.65962 | | 10.0851 | | 2.74 | | 0.0061 |
| c0 | -0.02461 | | 0.00124 | | -19.77 | | <.0001 |
| c1 | 0.000431 | | 0.000256 | | 1.68 | | 0.0923 |
| c2 | 0.001127 | | 0.000235 | | 4.80 | | <.0001 |
| c4 | 0,00051 | | 0.000232 | | 2.20 | | 0.0280 |
| c5 | 0.000338 | | 0.000220 | | 1.53 | | 0.1255 |
| c6 | 0.000178 | | 0.000223 | | 0.80 | | 0.4259 |
| c7 | -0.0003 | | 0.000456 | | -0.67 | | 0.5036 |
| c8 | 0.004014 | | 0.000301 | | 13.34 | | <.0001 |
| c9 | 0.000865 | | 0.000121 | | 7.13 | | <.0001 |
| c11 | 0.014749 | | 0.00147 | | 10.01 | | <.0001 |

**Example 11: Dressing Percentage Model**

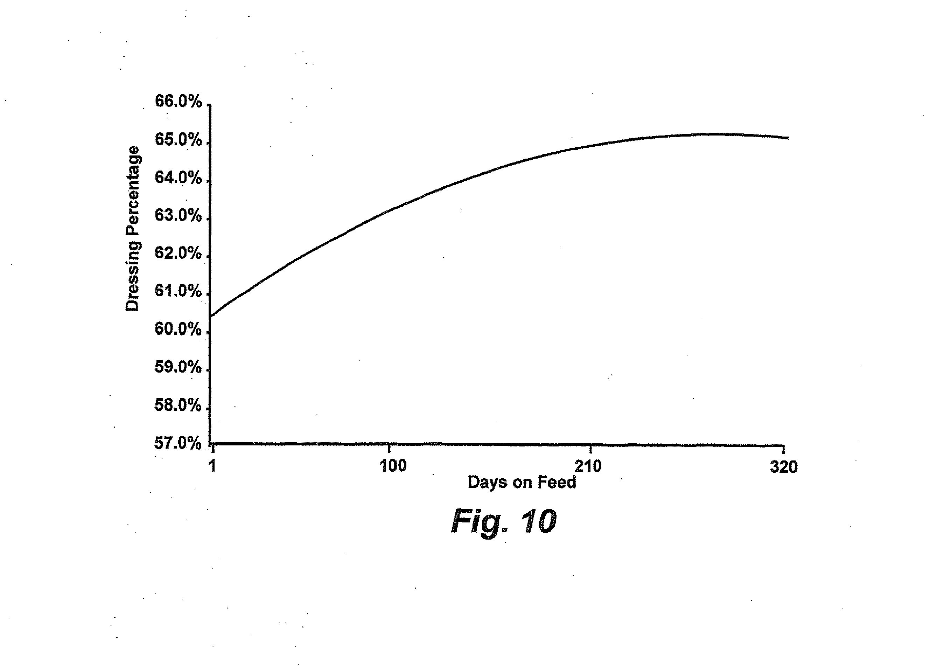

Unlike the dependent variables in the previous models, the data set did not contain serial measures of dressing percent. Therefore, a simple OLS model is estimated where dressing percentage is estimated as a function of placement weight, ultrasound backfat at placement, gender, genotype, days on feed, and days on feed squared. Estimation results are shown in Table 15 and Fig. 10.

## EP 2 468 900 A2

*Table 15*

| Dependent Variable: DP DP | | | | | | |
| Number of Observations Read: 1653 | | | | | | |
| Analysis of Variance | | | | | | |

| Source | DF | | Sum of Squares | Mean Square | F Value | Pr > F |
|---|---|---|---|---|---|---|
| Model | 13 | | 592.33853 | 45.56450 | 11.76 | <.0001 |
| Error | 1639 | | 6349.78039 | 3.87418 | | |
| Corrected Total | 1652 | | 6942.11891 | | | |
| Root MSE | | 1.96829 | | R-Square | 0.0853 | |
| Dependent Mean | | 63.51977 | | Adj R-Sq | 0.0781 | |
| Coeff Var | | 3.09871 | | | | |

| Parameter Estimates | | | | | | |
|---|---|---|---|---|---|---|
| | | | | Parameter | | Standard |
| Variable | Label | DF | Estimate | Error | t Value | Pr > |t| |
| Intercept | Intercept | 1 | 58.45820 | 1.79835 | 32.51 | <.0001 |
| wt1 | wt1 | 1 | 0,00282 | 0.0007182 | 3.92 | <.0001 |
| BFAT1 | BFAT1 | | 6.53002 | 1.87074 | 3.49 | 0.0005 |
| Frame1 | Frame1 | 1 | -0.13363 | 0.05731 | -2.33 | 0.0198 |
| steer | | 1 | -0.64269 | 0.18049 | -3.56 | 0.0004 |
| method1b | | 1 | 0.12170 | 0.16525 | 0.74 | 0.4616 |
| gen1 | | 1 | 0.45431 | 0.25024 | 1.82 | 0.0696 |
| gen2 | | 1 | 0.26332 | 0.21508 | 1.22 | 0.2210 |
| gen4 | | 1 | 0.49219 | 0.20467 | 2.40 | 0.0163 |
| gen5 | | 1 | 0.09207 | 0.20224 | 0.46 | 0.6490 |
| gen6 | | 1 | 0.14125 | 0.21080 | 0.67 | 0.5029 |
| gen7 | | 1 | -0.52955 | 0.40743 | -1.30 | 0.1939 |
| DOF | DOF | 1 | 0.03406 | 0.02228 | 1.53 | 0.1266 |
| dof2 | | 1 | -0.0000594 | 0.000073 | -0.81 | 0.4187 |

**Example 12: Dry Matter Intake Model**

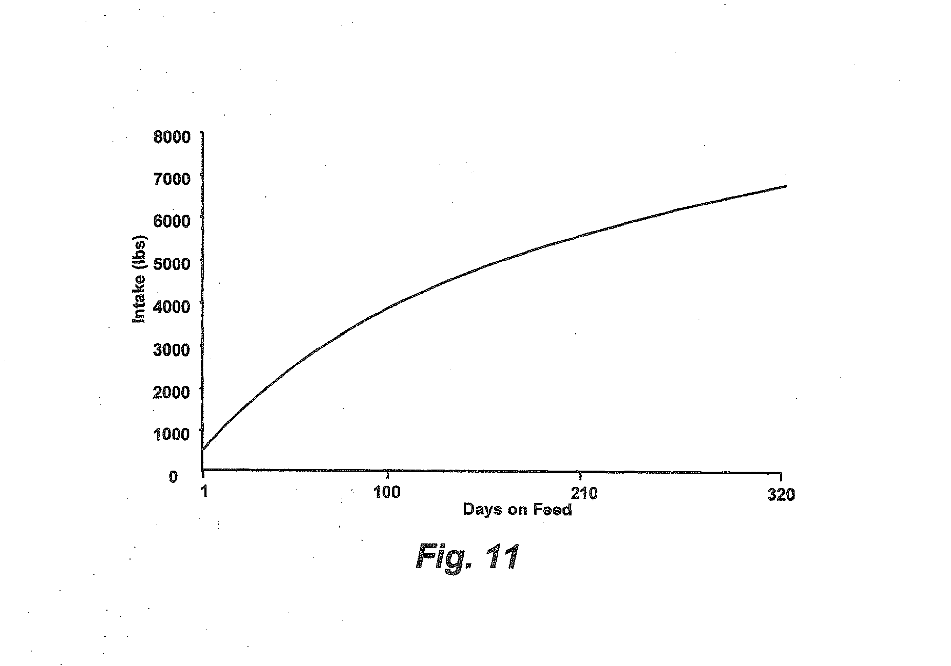

The data set does not contain serial measures of DMI. Therefore, a simple OLS model was estimated where DMI was estimated as a function of placement weight, ultrasound backfat at placement, gender, genotype, days on feed, and days on feed squared, live weight, and live weight squared as shown in Table 16 and Fig. 11.

*Table 16*

| Dependent Variable: DMI DMI; Number of Observations Used, 1653 | | | | | |
|---|---|---|---|---|---|
| Analysis of Variance | | | | | |
| Source | DF | Sum of Squares | Mean Square | F Value | Pr > F |
| Model | 15 | 330208299 | 22013887 | 560.78 | <.0001 |
| Error | 1637 | 64261353 | 39256 | | |
| Corrected Total | 1652 | 394469652 | | | |
| Root MSE | 198.13016 | R-Square | 0.8371 | | |
| Dependent Mean | 2953.70033 | Adj R-Sq | 0.8356 | | |
| Coeff Var | 6.70786 | | | | |

(continued)

| Dependent Variable: DMI DMI; Number of Observations Used, 1653 | | | | | | |
|---|---|---|---|---|---|---|
| | | Analysis of Variance | | | | |
| Source | DF | Sum of Squares | Mean Square | F Value | Pr > F | |
| | | Parameter Estimates | | | | |
| Variable | Label | DF | Parameter Estimate | Error | standard t Value | Pr> \|t\| |
| Intercept | Intercept | 1 | -4160.81922 | 411.47431 | -10.11 | <.0001 |
| gen.1 | | 1 | 12.09223 | 25.20955 | 0.48 | 0.6315 |
| gen2 | | 1 | 4.10949 | 21.69136 | 0.19 | 0.8498 |
| gen4 | | 1 | 47.11832 | 20.62314 | 2.28 | 0.0225 |
| gen5 | | 1 | 29.39816 | 20.38129 | 1.44 | 0.1494 |
| gen6 | | 1 | 49.74598 | 21.23470 | 2.34 | 0.0193 |
| gen7 | | 1 | 57.85811 | 41.03954 | 1.41 | 0.1588 |
| steer | | 1 | -136.16122 | 18.32428 | -7.43 | <.0001 |
| method1b | | 1 | 1.29215 | 16.65221 | 0.08 | 0.9382 |
| | | 1 | -3.07766 | 0.11411 | -26.97 | <.0001 |
| wt1 | wt1 | 1 | 50.93323 | 5.94744 | 8.56 | <.0001 |
| Frame1 | Frame1 | 1 | 747.73848 | 195.20756 | 3.83 | 0.0001 |
| BEAT1 | BFAT1 | 1 | 6.93798 | 0.63617 | 10.91 | <.0001 |
| Calc_Lv | Calc Lv | 1 | -0.00101 | 0.0002589 | -3.89 | 0.0001 |
| Wt | Wt | 1 | | | | |
| wtf2 | | 1 | 23.07109 | 2.25140 | 10.25 | <.0001 |
| DOF | DOF | | -0.06336 | 0.00740 | -8.57 | <.0001 |
| dof2 | | | | | | |

## Example 13: Ribeye Area Model

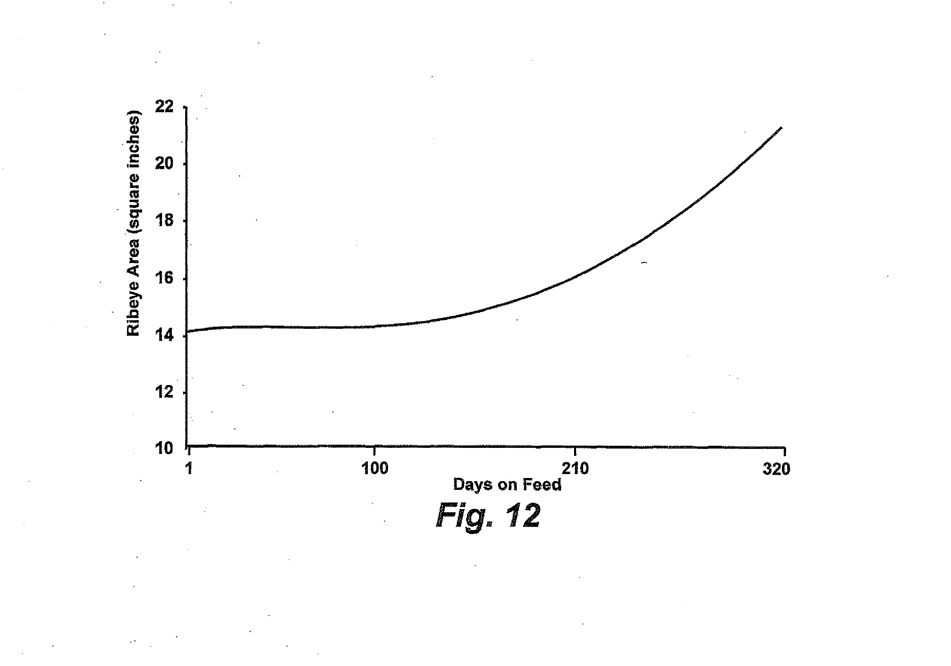

The data set does not contain serial measures of ribeye area. Therefore, a simple OLS model was estimated where ribeye rea was estimated as a function of placement weight, ultrasound backfat at placement, gender, genotype, days on feed, and days on feed squared, live weight, and live weight squared as shown in Table 17 and Fig. 12.

*Table 17*

| Model: MODEL1 | | | | | |
|---|---|---|---|---|---|
| Dependent Variable: REA REA | | | | | |
| Number of Observations Read 1653 | | | | | |
| Number of Observations Used 1653 | | | | | |
| Analysis of Variance | | | | | |
| Source | DF | Sum of Squares | Mean Square | F Value | Pr > F |
| Model | 15 | 958.96864 | 63.93124 | 28.51 | <.0001 |
| Error Corrected | 1637 | 3670.21431 | 2.24204 | | |
| Total | 1652 | 4629.18295 | | | |
| Root MSE | 1.49734 | R-Square | 0.2072 | | |
| Dependent Mean | 13.48881 | | | | |
| Coeff Va | 11.10063 | Adj R-Sq | 0.1999 | | |

(continued)

| Parameter Estimates | | | | | | |
|---|---|---|---|---|---|---|
| | | | | | Parameter Standard | |
| Variable | Label | DF | Estimate | Error | t Value | Pr > \|t\| |
| Intercept | Intercept | 1 | 3.17944 | 3.10966 | 1.02 | 0.3067 |
| gen1 | | 1 | 0.34357 | 0.19052 | 1.80 | 0.0715 |
| gen2 | | 1 | 0.11909 | 0.16393 | 0.73 | 0.4676 |
| gen4 | | 1 | 0.33572 | 0.15586 | 2.15 | 0.0314 |
| gen5 | | 1 | -0.16206 | 0.15403 | -1.05 | 0.2929 |
| gen6 | | 1 | -0.13984 | 0.16048 | -0.87 | 0.3837 |
| gen7 | | 1 | -0.34558 | 0.31015 | -1.11 | 0.2653 |
| steer | | 1 | -0.05313 | 0.13848 | -0.38 | 0.7013 |
| method1b | | 1 | -0.33554 | 0.12585 | -2.67 | 0.0077 |
| wt1 | wt1 | 1 | 0.00458 | 0.00086238 | 5.31 | <.0001 |
| Frame1 | Frame1 | | -0.06462 | 0.04495 | -1.44 | 0.1507 |
| BFAT1 | BFAT1 | 1 | -5.43485 | 1.47526 | -3.68 | 0.0002 |
| Catc_Lv _Wt | Calc Lv Wt | 1 | 0.01438 | 0.00481 | 2.99 | 0.0028 |
| wtf2 | | 1 | -0.0000049 | 0.000001 | -2.54 | 0.0111 |
| DOF | DOF | | -0.03529 | 0.01701 | -2.07 | 0.0382 |
| dof2 | | 1 | 0.000152 | 0.00005 | 2.72 | 0,0065 |

## Example 14: Prediction of Profit per Head by Marketing Method and Genotype at Optimal Days on feed

The profit per head of animal for each possible ob gene genotype and marketing method is shown in Table 18. In this simulation, the grid method of marketing is the optimum regardless of the genotype. If the independent factor is the marketing method, then the optimum genotype is E-CC/U-CC other than the grid system, where "other" genotype is preferred. Exemplary profit per head of cattle is also given in Table 18.

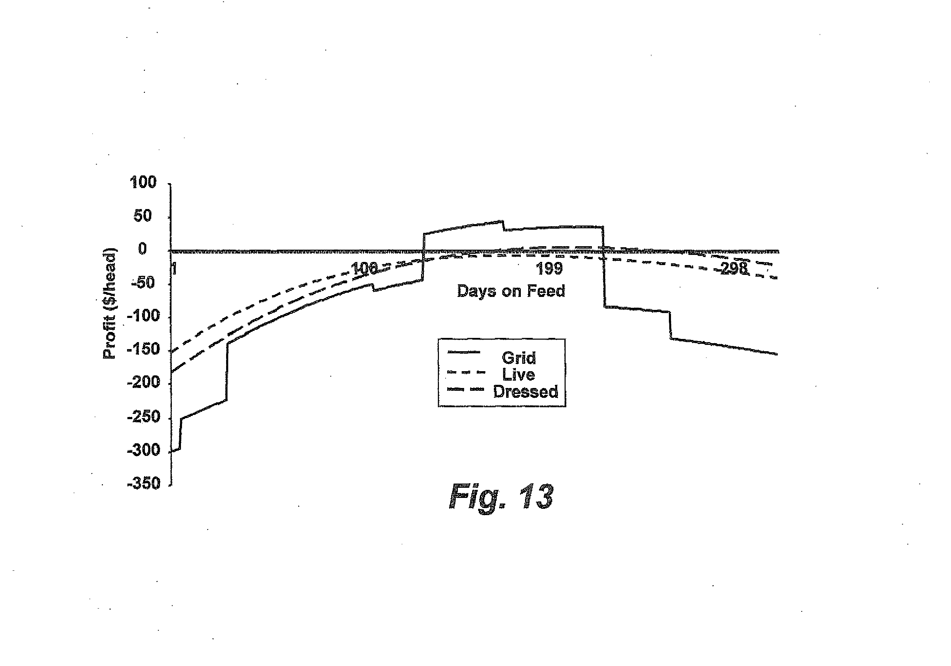

Fig. 13 shows the predicted outcomes for the three marketing methods of live weight, dressed and grid for a group of E-CC/U-CC cattle in a simulated feeding. To obtain this graphical representation, the predicted changes in the backfat, marbling score, live weight, dressing percentage, ribeye area and yield grade over a total feeding time of 325 days was calculated according to the equations presented in Examples 7 to 12 above. The value for each trait at each day of feeding was then used together with the price adjustments (premiums and discounts) as shown, for example, in Table 4 above, to determine the grid price at each day of feeding. Also calculated were the expected live weight prices and the dressed weight prices at each time point. The results were plotted as shown in Fig. 13. In this simulation, for a group of cattle having the E-CC/U-CC genotype, a profit per head of cattle is realized for animals fed during the 133 to 230 feeding period if marketed by the grid method of price determination. A smaller profit margin is realized for the dressed marketing method for animals fed for 162 to 265 days, and no profit for animals marketed by the live weight method.

*Table 18*

| | Genotype | | | | |
|---|---|---|---|---|---|
| *Marketing Method* | E-cc\|U-cc | E-cc\|U-ct | E-cc\|U-tt | E-ct\|U-cc | E-ct\|U-ct |
| Live Weight ($/head) | -$6.58 | -$12.31 | -$57.04 | -$33.83 | -$37.44 |
| Dressed Weight ($/head) | $5.86 | -$2.73 | -$58.87 | -$23.88 | -$34.83 |
| Grid ($/head) | $44.13 | $36-59 | $46.43 | $50.44 | $43.96 |

(continued)

| Marketing Method | Genotype | | | | |
|---|---|---|---|---|---|
| | E-cc\|U-cc | E-cc\|U-ct | E-cc\|U-tt | E-ct\|U-cc | E-ct\|U-ct |
| **SUMMARY Optimal Marketing Method for Genotype** | | | | | |
| E-cc/U-cc | Grid | | | | |
| E-cc/U-ct | Grid | | | | |
| E-cc/U-tt | Grid | | | | |
| E-ct/U-cc | Grid | | | | |
| E-ct/U-ct | Grid | | | | |
| E-tt/U-cc | Grid | | | | |
| other | Grid | | | | |
| **Optimal Genotype by Marketing Method** | | | | | |
| Live Weight | E-cc\|U-cc | | | | |
| Dressed Weight Grid 1 | E-cc\|U-cc other | | | | |
| Value difference ($/head) between genotype with highest revenue and other genotypes when marketing by **Live** basis | | | | | |
| E-cc\|U-cc | $0.00 | | | | |
| E-cc\|U-ct | $5.72 | | | | |
| E-cc\|U-tt | $50.46 | | | | |
| E-ct\|U-cc | $27.24 | | | | |
| E-ct\|U-ct | $30.86 | | | | |
| E-tt\|U-cc | $35.56 | | | | |
| other | $45.15 | | | | |
| Value difference ($/head) between genotype with highest revenue and other genotypes when marketing by **Dressed** Weight basis | | | | | |
| E-cc\|U-cc | $0.00 | | | | |
| E-cc\|U-ct | $8.59 | | | | |
| E-cc\|U-tt | $64.73 | | | | |
| E-ct\|U-cc | $29.74 | | | | |
| E-ct\|U-ct | $40.69 | | | | |
| E-tt\|U-cc | $44.50 | | | | |
| other | $65.00 | | | | |
| Value difference ($/head) between genotype with highest revenue and other genotypes when marketing by **Grid** basis | | | | | |
| E-cc\|U-cc | $15.19 | | | | |
| E-cc\|U-ct | $22.73 | | | | |
| E-cc\|U-tt | $12.89 | | | | |
| E-ct\|U-cc | $8.88 | | | | |
| E-ct\|U-ct | $15.36 | | | | |
| E-tt\|U-cc | $13.03 | | | | |
| other | $0.00 | | | | |

While the invention has been described with reference to specific methods and embodiments, such description is for illustrative purposes only. The words used are words of description rather than of limitation. It is to be understood that changes and variations may be made by those of ordinary skill in the art without departing from the spirit or scope of the present invention, which is set forth in the following claims.

*SUMMARY PARAGRAPHS*

The present invention is defined in the claims and the accompanying description. For convenience other aspects of the present invention are presented herein by way of numbered paragraphs (para. s).

1. A computer-assisted method for determining revenue from a cattle marketing method comprising using a programmed computer comprising a processor, a data storage system, an input device and an output device, and the steps of: (a) determining the genotype of an animal or group of animals by identifying at least two single length polymorphisms of the animal or animals; (b) inputting data into the programmed computer through the input device, wherein the data comprises a genotype of an animal, a physical characteristic of the animal at placement, a carcass prediction and a plurality of predefined market prices; (c) calculating a revenue expectation from a cattle marketing method for a plurality of genotypes by calculating a live weight price, a dressed weight price, and a grid price for each genotype, wherein the grid price comprises premium and discount prices; (d) correlating the expected revenues with the genotypes and the marketing methods; and (e) outputting to the output device the expected revenues for the cattle marketing methods.

2. The method according to Paragraph 1, further comprising the step of identifying the marketing method providing the highest revenue for an animal or group of animals.

3. The method according to Paragraph 1, further comprising the step of identifying the marketing method providing the highest revenue for a genotype.

4. The method according to Paragraph 1, wherein the input data is selected from a genotype, placement weight, ultrasound backfat measurement at placement, frame score at placement, days on feed, and gender

5. The method according to Paragraph 1, further comprising the steps of: (a) calculating the predicted values of the yield grade, the quality grade, and the dressing percentage of an animal for the cattle marketing method by using at least one estimated prediction equation defining the change in a trait of an animal over a period of feeding; (b) calculating the expected revenues for live weight, dressed weight and grid marketing methods by using the predicted values of yield grade, the quality grade, and the dressing percentage; (c) determining the marketing method giving the highest expected revenue for the animal.

6. The method according to Paragraph 5 wherein the trait of an animal is selected from the group of traits consisting of backfat production, marbling score, weight prediction, dressing percentage, dry matter intake and rib eye area.

7. The method according to Paragraph 5, wherein step (a) comprises using a plurality of prediction equations, wherein the predictive equations determines the changes in a plurality of traits of an animal over a period of feeding.

8. The method according to Paragraph 1, wherein the genotype of the animal is determined from at least two single-length polymorphisms of the *ob* gene.

9. The method according to Paragraph 8, wherein the single-length polymorphisms are UASMS2 and EXON2-FB of the ob gene.

10. The method according to Paragraph 1, further comprising the step of marketing the animal by the marketing method that produces the greatest expected revenue.

11. The methods of paragraphs 2 and 3 wherein the genotype is an *ob* genotype,

12. The method of paragraph 4 wherein the physical characteristic correlating to a CC genotype is a low propensity to deposit fat.

13. The method of paragraph 4 wherein the physical characteristic correlating to a TT genotype is a high propensity to deposit fat.

14. The method of paragraph 4 wherein the physical characteristic correlating to a CT genotype is an intermediate propensity to deposit fat.

15. A method of transmitting data comprising transmission of information from the methods according to Paragraph 1 via telecommunication, telephone, video conference, mass communication, presentation graphics, internet, email, or paper or electronic documentary communication.

16. A computer-assisted method for determining revenue from a cattle marketing method comprising using a programmed computer comprising a processor, a data storage system, an input device and an output device, and the steps of: (a) determining the genotype of an animal or group of animals by identifying the haplotype of the animal or animals, wherein single-length polymorphisms defining the haplotype are UASMS2 and EXON2-FB of the ob gene; (b) inputting data into the programmed computer through the input device, wherein the data comprises a genotype of an animal and at least one of a physical characteristic of the animal at placement, a carcass prediction and a plurality of predefined market prices, genotype, placement weight, ultrasound backfat measurement at placement, frame score at placement, days on feed, and gender; (c) calculating a revenue expectation from a cattle marketing method for a plurality of genotypes by calculating a live weight price, a dressed weight price, and a grid price for each genotype, wherein the grid price comprises premium and discount prices; (d) correlating the expected revenues with the genotypes and the marketing methods; (e) calculating the predicted values of the yield grade, the quality grade, and the dressing percentage of an animal for the cattle marketing method by using a plurality of estimated prediction equations, wherein the predictive equations determine the changes in a plurality of traits of an animal over a period of feeding, and wherein the traits of an animal is selected from the group of traits consisting of backfat production, marbling score, weight prediction, dressing percentage, dry matter intake and rib eye area; (f) calculating the expected revenues for live weight, dressed weight and grid marketing methods by using the predicted values of yield grade, the quality grade, and the dressing percentage; (g) determining the marketing method giving the highest predicted expected revenue for the animal (h) identifying the marketing method providing the highest revenue for an animal or group of animals; (i) outputting to the output device the expected revenues for the cattle marketing methods; and (j) marketing each animal by the marketing method that produces the greatest expected revenue.

**Claims**

1. A method for identifying a bovine animal having a higher or lower Placement Weight (PW), a higher Ultrasound Backfat at placement (BF), a higher or lower Percent Steer (PS), a higher or lower Marbling Score (MBS), a higher or lower Calculated Yield Grade (CYG), a higher or lower Plant Backfat (PB), and a higher or lower Live Weight at Slaughter (LWS); and a lower or higher Frame Score at placement (FS), a lower or higher number of Days on Feed (DF), and a lower or higher Dressing Percentage (DP), as compared to a general population of bovine animals, said method comprising:

   (a) obtaining a biological sample from said bovine animal, said biological sample comprising nucleic acids encoding the bovine leptin gene from said bovine;
   (b1) detecting in said nucleic acids the presence of at least one the following combinations of an EXON2-FB (E) / UASMS2 (U) genotype: E=TT and U= CT, E=TT and U=TT, and E=CT and U=TT; and correlating the presence of the nucleic acid content of (b1) with a higher PW, BF, PS, MBS, CYG, PB, and LWS; and a lower FS, DF, and DP; thereby identifying the bovine animal; or
   (b2) detecting in said nucleic acids the presence of at least one the following combinations of an EXON2-FB (E) / UASMS2 (U) genotype: E=CT and U= CT or E=TT and U=CC; and correlating the presence of the nucleic acid content of (b2) with a lower PW, BF, PS, MBS, CYG, PB, and LWS; and a higher FS, DF, and DP; thereby identifying the bovine animal.

2. The method of claim 1 further comprising the step of determining whether the bovine animal should be marketed on the basis of Dressed Weight or Live Weight in order to obtain greater revenue per bovine animal.

3. The method of claim 2 wherein bovine animals having the E=CC / U=CC, E=CC / U=CT, or E=CT / U=CC genotype are marketed on the basis of Dressed Weight method, and bovine animals having all other E / U genotypes are marketed on the basis of Live Weight, in order to obtain greater revenue per bovine animal.

4. The method of claim 3 wherein bovine animals having the E=CC / U=CC genotype generate higher revenue, as compared to a general population of bovine animals, and wherein bovine animals having the E=CC / U=TT generate a lower revenue, as compared to a general population of bovine animals.

5. The method of claim 3 wherein greater revenue is generated per bovine animal based upon the following rank order of genotypes: E=CC / U=CC > E=CT / U=CC > E=CC / U=CT > E=TT / U=CC > E=CT / U=CT > E=CC / U=TT.

6. The method of any one of claims 1 to 3 which further comprises inputting into a computer bovine genotype data, optionally including whether the bovine should be marketed on the basis of Dressed Weight or Live Weight, to assist an owner of cattle in obtaining the greatest revenue for the bovine animals.

Fig. 1

*Fig. 2*

*Fig. 3*

EP 2 468 900 A2

*Fig. 4*

*Fig. 5*

*Fig. 6*

EP 2 468 900 A2

Fig. 7

*Fig. 8*

EP 2 468 900 A2

*Fig. 9*

Fig. 10

EP 2 468 900 A2

EP 2 468 900 A2

*Fig. 11*

Fig. 12

*Fig. 13*

REFERENCES CITED IN THE DESCRIPTION

**Patent documents cited in the description**

- US 89454607 A
- US 4683195 A
- US 4683202 A
- US 6569672 B
- US 6569627 B
- US 6562298 B
- US 6556940 B
- US 6489112 B
- US 6482615 B
- US 6472156 B
- US 6413766 B
- US 6387621 B
- US 6300124 B
- US 6270723 B
- US 6245514 B
- US 6232079 B
- US 6228634 B
- US 6218193 B
- US 6210882 B
- US 6197520 B
- US 6174670 B
- US 6132996 B
- US 6126899 B
- US 6124138 B
- US 6074868 B

- US 6036923 B
- US 5985651 B
- US 5958763 B
- US 5942432 B
- US 5935522 B
- US 5897842 B
- US 5882918 B
- US 5840573 B
- US 5795784 B
- US 5795547 B
- US 5785926 B
- US 5783439 B
- US 5736106 B
- US 5720923 B
- US 5720406 B
- US 5675700 B
- US 5616301 B
- US 5576218 B
- US 5455175 B
- US 891256 A
- US 061942 A
- US 6548256 B
- US 5989431 B

**Non-patent literature cited in the description**

- **ZHANG et al.** *Nature,* 1994, vol. 372, 425-432
- **JI et al.** *Anim. Biotech.,* 1998, vol. 9, 1-14
- **HOUSEKNECHT et al.** *J. Anim. Sci.,* 1998, vol. 76, 1405-1420
- **LORD et al.** *Nature,* 1998, vol. 394, 897-901
- **WILLIAMS et al.** *Domest. Anim. Endocrinol.,* 2002, vol. 23, 339-349
- **LIEFERS et al.** *J. dairy Sci.,* 2002, vol. 85, 1633-1638
- **LAGONIGRO et al.** *Anim. Genet.,* 2003, vol. 34, 371-374
- **BUCHANAN et al.** *Genet Sel. Evol.,* 2002, vol. 34, 105-116
- **BUCHANAN et al.** *Genet Sel Evol.,* 2002, vol. 34, 105-16

- **HIROTA et al.** *Jinrui Idengaku Zasshi.,* 1989, vol. 34, 217-23
- **JOHN et al.** *Nucleic Acids Res.,* 1991, vol. 19, 408
- **VIGNAL et al.** *Genet. Sel. Evol.,* 2002, vol. 34, 275-305
- **KAJIMURA et al.** *GATA,* 1990, vol. 7 (4), 71-79
- **BRETHOUR.** *J. Anim. Sci.,* 2000, vol. 78, 2055-61
- **BRETHOUR.** *J. An. Sci,* 2000
- **KAPS et al.** *J. Anim. Sci.,* 1999, vol. 77, 569-74